# EUROPEAN PATENT APPLICATION

(11) **EP 2 623 502 A1**
(43) Date of publication of application: **07.08.2013**
(21) Application number: 11829366.1
(22) Date of filing: 30.09.2011
(51) Int. Cl.: C07D 307/60, A01N 43/08, A01P 13/02, A01P 21/00, C07D 405/12

(54) **NOVEL COMPOUND HAVING STRIGOLACTONE-LIKE ACTIVITY AND USE THEREOF**

(30) Priority: 30.09.2010 JP 2010221766
(71) Applicant: Riken, Saitama 351-0198 (JP)
(72) Inventor: ASAMI Tadao, Tokyo 162-0855 (JP); ITO Shinsaku, Tokyo 114-0024 (JP); FUKUI Kosuke, Tokyo 113-0021 (JP); NAKANO Takeshi, Wako-shi Saitama 351-0198 (JP)
(74) Representative: Woods, Geoffrey Corlett
(86) International application number: PCT/JP2011/072607
(87) International publication number: WO 2012/043813

(57) **Abstract**

The present invention provides a compound that has an activity equal to or higher than that of natural strigolactone, can be chemically synthesized by a convenient step and at low cost, exhibits stability in environments, and has no or weak seed germination-promoting effect on plants of the genus *Striga* or *Orobanche.* Specifically, the present invention provides a compound represented by the general formula (III) or a salt thereof, wherein X represents a lower alkyl group or the like; Q represents a single bond or the like; and ring C represents a 6-membered ring represented by the formula (IVa) or the like wherein R1, R2, R3, and R4 each independently represent a carbon atom or the like, Z1 and Z2 are bonded to the ring atoms of ring C and each independently represent a hydrogen atom, a halogen atom, or the like, and the groups R5, R6, R7, Z3, Z4, and Z5 are as defined in the specification.

## Description

### Technical Field

The present invention relates to a novel compound and use thereof. Specifically, the present invention relates to a compound having a strigolactone-like activity and a method for producing the same. The present invention also relates to a method for inhibiting plant branching or controlling a root parasitic plant using the compound, and an agricultural chemical composition comprising the compound.

### Background Art

The regulation of plant branching is important for, for example, controlling the yields of agricultural and horticultural crops. Hence, in the agricultural and forestry fields, there is a demand for an efficient, safe, and inexpensive branching-regulating agent.

Cytokinins (trans-zeatin and its derivatives) and auxins (indoleacetic acid and its derivatives) are known as important hormones for regulating the branching patterns of plants. The cytokinins, which are hormones promoting the growth of axillary buds, are known to be biosynthesized locally and transiently in stems for the breaking of dormancy of axillary buds and the subsequent outgrowth, and supplied to the axillary buds to promote axillary bud outgrowth. By contrast, the auxins are biosynthesized in apical buds. The biosynthesized hormones are basipetally transferred in stems via an auxin transporter protein PIN to inhibit the growth of axillary buds in the plant. Thus, the auxins may serve as plant branching inhibitors. The auxins, however, exhibit not only the effect of inhibiting axillary bud growth, but also various effects such as a cell division-promoting effect, a growth-promoting effect, an ovary growth-promoting effect, and a rooting-promoting effect. Thus, the hormones, when externally administered, may disadvantageously cause plant deformity. Hence, the auxins are not suitable as practical branching inhibitors. Heretofore, an auxin antagonist maleic acid hydrazide has been used as an agricultural chemical for inhibition of tobacco axillary buds. Use of this agricultural chemical, however, is currently regulated in terms of safety, due to the undesired carcinogenicity of hydrazide mixed therein as an impurity.

Meanwhile, a terpenoid compound strigolactone is a newly found plant hormone, and its diverse applicability to the regulation of plant morphogenesis, etc., has been reported (Patent Literature 1 and Non Patent Literatures 1 to 6). Natural strigolactone, however, is valuable and hardly available. In general, natural strigolactone and its derivatives must be chemically synthesized through many complicated reaction steps (Non Patent Literatures 1 to 6). Hence, the natural strigolactone, albeit highly active, is difficult to synthesize at a large scale in relation to production cost and synthesis time and is thus disadvantageously impractical.

Also, the natural strigolactone and its derivatives are known to mostly have the effect of promoting the seed germination of root parasitic plants of the genus *Striga* or *Orobanche* (Non Patent Literature 7). These root parasitic plants grow by taking the roots of host plants and depriving them of nutrients and moisture, because these plants are not capable of photosynthesis required for acquiring energy necessary for their own survival and growth or have no chlorophyll. These root parasitic plants significantly inhibit the growth of host plants through their parasitism and therefore inflict enormous damage on agricultural production around the world. Thus, the application of strigolactone or its derivative to a field where host plants are cultivated includes the risk of inducing the seed germination of such root parasitic plants and affecting the agricultural production itself. On the contrary, the root parasitic plants cannot grow without parasitizing host plants, as indicated by the name. In this regard, the application of strigolactone or its derivative to a field before cultivation of host plants can induce suicidal germination, which causes the forced germination and killing of the root parasitic plants.

A large majority of previously reported natural and non-natural strigolactones have an enol ether bond susceptible to decomposition. This structure is regarded as an essential structure for exhibiting a strigolactone-like activity. Unfortunately, these compounds are low stable in environments and difficult to practically apply, due to the presence of this enol ether bond.

### Citation List

### Patent Literature

Patent Literature 1: WO 2008/152092

### Non Patent Literature

Non Patent Literature 1: Kondo Y, et al., Biosci Biotech Biochem, 71 (11): 2781-2786, 2007
Non Patent Literature 2: Mangnus EM and Zwanenburg B, J. Agric Food Chem, 40 (6): 1066-1070, 1992
Non Patent Literature 3: Nefkens GHL, et al., J Agric Food Chem, 45 (6): 2273-2277, 1997
Non Patent Literature 4: Galindo JC, et al., J Agric Food Chem, 50 (7): 1911-1917, 2002
Non Patent Literature 5: Zwanenburg B, et al., Pest Manag Sci, 65 (5): 478-491, 2009
Non Patent Literature 6: Akiyama K, et al., Plant Cell Physiol, 51 (7): 1104-1117, 2010
Non Patent Literature 7: Cook et al., Journal of the American Chemical Society, 94: 6198-6199, 1972

### Summary of Invention

### Technical Problem

There has been a demand for the development of a compound that has an activity equal to or higher than that of natural strigolactone, can be chemically synthesized by a convenient step and at low cost, exhibits stability in environments, and has no or weak seed germination-promoting effect on root parasitic plants.

### Solution to Problem

The present inventor has conducted diligent studies to solve the problems described above and consequently found compounds that have a novel skeleton, can be synthesized easily at low cost through a reaction pathway as short as one stage or two stages, and have a high strigolactone-like activity and high stability in environments. Some of the compounds are excellent in plant branching inhibitory activity and also have low germination-inducing activity against root parasitic plants *Orobanche minor* and *Striga hermonthica.* Thus, the present inventor has also found that these compounds can be used as plant branching inhibitors that do not induce the germination of such root parasitic plants. In addition, some of the compounds have poor plant branching inhibitory activity and also have high germination-inducing activity against root parasitic plants. Thus, the present inventor has further found that these compounds can induce the suicidal germination of such root parasitic plants. The present invention has been made on the basis of these findings and specifically includes the followings:

[1] A compound represented by the following general formula (III) or a salt thereof:

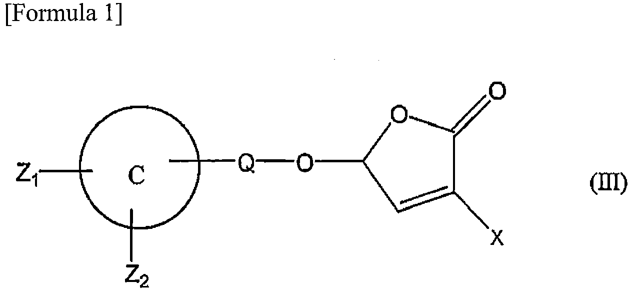

wherein
X represents a lower alkyl group, a lower alkenyl group, a lower alkynyl group, a halogen atom, an OH group, a CN group, a NO₂ group, a CF₃ group, a carboxyl group, or a carboxyl group substituted by a lower alkyl group or a halogen atom;
Q represents a single bond, -CO-, or -CH₂-; and
ring C represents a 6-membered ring represented by the following formula (IVa) or (IVb) or a 5-membered ring represented by the following formula (IVc) or (IV d):

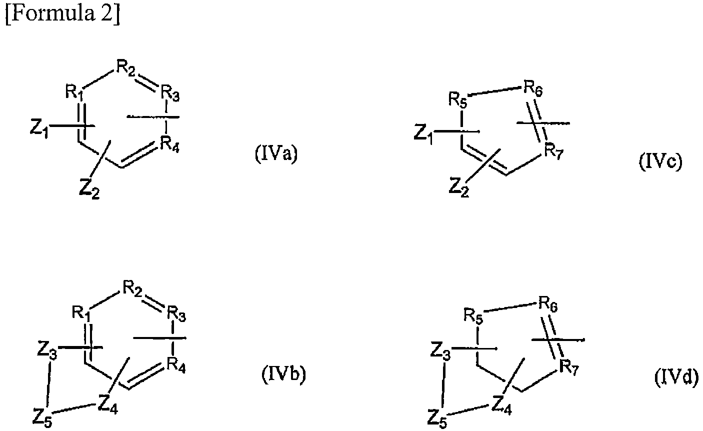

wherein R1, R2, R3, and R4 in the formulas (IVa) and (IVb) each independently represent a carbon atom optionally bonded to a halogen atom or an oxygen atom, a nitrogen atom, a sulfur atom, or an oxygen atom,
R5, R6, and R7 in the formulas (IVc) and (IVd) each independently represent a carbon atom optionally bonded to a halogen atom or an oxygen atom, a nitrogen atom, a sulfur atom, or an oxygen atom,
Z1 and Z2 in the formulas (IVa) and (IVc) are bonded to the ring atoms of ring C and each independently represent a hydrogen atom, a halogen atom, a NO₂ group, a CF₃ group, a CN group, an alkyl group, an alkoxy group, a carboxyl group, a carboxyl group substituted by a lower alkyl group or a halogen atom, an aryl group, an aryloxy group, or an aryl group or an aryloxy group substituted by lower alkyl or a halogen atom,
Z3 and Z4 in the formulas (IVb) and (IVd) each independently represent a carbon atom, a nitrogen atom, a sulfur atom, or an oxygen atom bonded to Z5 to form a 5-membered ring or a 6-membered ring, and Z5 is bonded to Z3 and Z4 to form the 5-membered ring or the 6-membered ring and represents a group constituted of atoms selected from the group consisting of a carbon atom, a nitrogen atom, a sulfur atom, and an oxygen atom.

[2] The compound according to [1] or a salt thereof, wherein Q is a single bond.

[3] The compound according to [1] or [2] or a salt thereof, wherein ring C is a 6-membered ring represented by the formula (IVa).

[4] The compound according to any of [1] to [3] or a salt thereof, wherein each of R1, R2, R3, and R4, or each of R5, R6, and R7 is a carbon atom or a carbon atom bonded to a halogen atom or an oxygen atom.

[5] The compound according to any of [1] to [4] or a salt thereof, wherein ring C is a ring represented by the formula (IVa) or (IVc) wherein Z1 is a hydrogen atom, a halogen atom, a NO₂ group, a CF₃ group, a methoxy group, a COOCH₃ group, a t-butyl group, or a CN group, and Z2 is a hydrogen atom.

[6] The compound according to any of [1] to [4] or a salt thereof, wherein ring C is an aryl group, an aryloxy group, or an aryl group or an aryloxy group substituted by lower alkyl or a halogen atom.

[7] The compound according to any of [1] to [4] or a salt thereof, wherein ring C is a ring represented by the formula (IVa) or (IVc) wherein Z1 and Z2 each independently represent a halogen atom, a CN group, or a CF₃ group.

[8] The compound according to any of [1] to [7] or a salt thereof, wherein X is a methyl group.

[9] The compound according to any of [1] to [8] or a salt thereof, wherein the compound or a salt thereof is a compound represented by the following general formula (V) or a salt thereof:

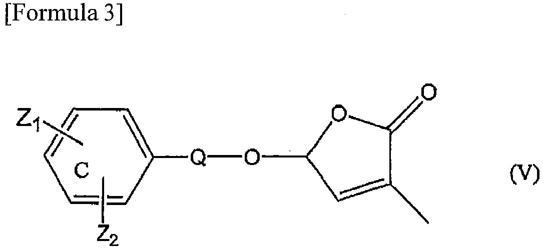

wherein Q represents a single bond or -CO-; and Z1 and Z2 are bonded to the carbon atoms of ring C and each independently represent a hydrogen atom, a halogen atom, a NO₂ group, a CF₃ group, a CN group, an alkyl group, an alkoxy group, a carboxyl group, or a carboxyl group substituted by a lower alkyl group or a halogen atom, or Z1 and Z2 form, together with ring C, an aryl group, an aryloxy group, or an aryl group or an aryloxy group substituted by lower alkyl or a halogen atom.

[10] A method for producing a compound represented by formula (III) or a salt thereof, the method comprising the step of reacting a ring C derivative represented by the following general formula (I):

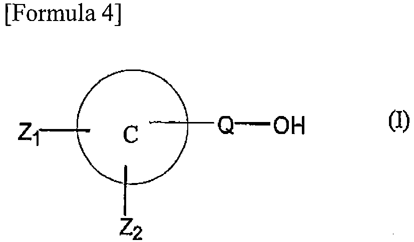

wherein
Q represents a single bond, -CO-, or -CH₂-, and
ring C represents a 6-membered ring represented by the following formula (IVa) or (IVb) or a 5-membered ring represented by the following formula (IVc) or (IVd):

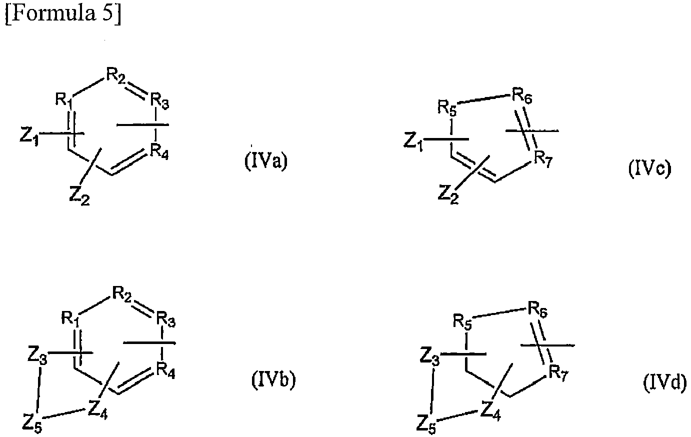

wherein R1, R2, R3, and R4 in the formulas (IVa) and (IVb) each independently represent a carbon atom optionally bonded to a halogen atom or an oxygen atom, a nitrogen atom, a sulfur atom, or an oxygen atom,
R5, R6, and R7 in the formulas (IVc) and (IVd) each independently represent a carbon atom optionally bonded to a halogen atom or an oxygen atom, a nitrogen atom, a sulfur atom, or an oxygen atom,
Z1 and Z2 in the formulas (IVa) and (IVc) are bonded to the ring atoms of ring C and each independently represent a hydrogen atom, a halogen atom, a NO₂ group, a CF₃ group, a CN group, an alkyl group, an alkoxy group, a carboxyl group, a carboxyl group substituted by a lower alkyl group or a halogen atom, an aryl group, an aryloxy group, or an aryl group or an aryloxy group substituted by lower alkyl or a halogen atom,
Z3 and Z4 in the formulas (IVb) and (IVd) each independently represent a carbon atom, a nitrogen atom, a sulfur atom, or an oxygen atom bonded to Z5 to form a 5-membered ring or a 6-membered ring, and Z5 is bonded to Z3 and Z4 to form the 5-membered ring or the 6-membered ring and represents a group constituted of atoms selected from the group consisting of a carbon atom, a nitrogen atom, a sulfur atom, and an oxygen atom,
with a brominated lactone represented by the following general formula (II):

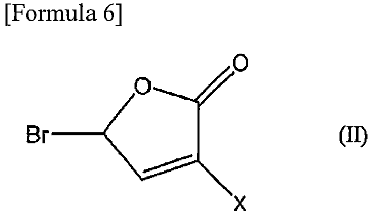

wherein
X represents a lower alkyl group, a lower alkenyl group, a lower alkynyl group, a halogen atom, an OH group, a CN group, a NO₂ group, a CF₃ group, a carboxyl group, or a carboxyl group substituted by a lower alkyl group or a halogen atom, under basic conditions to obtain a compound represented by the following formula (III):

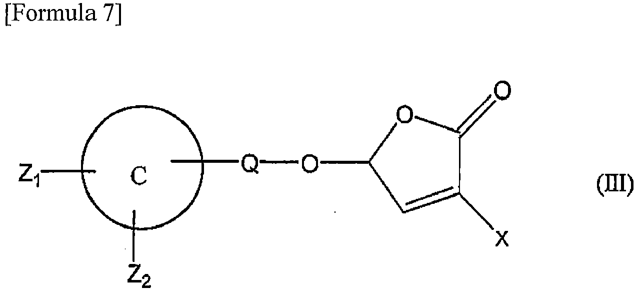

wherein X, Q, ring C, Z1, and Z2 are as defined above.

[11] An agricultural chemical composition comprising at least one compound according to any of [1] to [9] and/or a salt thereof and an agriculturally acceptable carrier.

[12] The agricultural chemical composition according to [11], wherein the agricultural chemical composition is a plant branching inhibitor.

[13] The agricultural chemical composition according to [12], wherein the plant is a crop plant or a horticultural plant.

[14] The agricultural chemical composition according to [11], wherein the agricultural chemical composition is a root parasitic plant control agent.

[15] The agricultural chemical composition according to [14], wherein the root parasitic plant is a plant belonging to the family *Orobanchaceae.*

[16] A method for inhibiting the branching of a plant, the method comprising the step of applying at least one compound according to any of [1] to [9] and/or a salt thereof to the plant or a medium or soil for growth of the plant.

[17] A method for controlling a root parasitic plant that parasitizes a plant, the method comprising the step of applying at least one compound according to any of [1] to [9] and/or a salt thereof to the plant or a medium or soil for growth of the plant.

### Advantageous Effects of Invention

The present invention provides novel compounds that have a strigolactone-like activity and can be chemically synthesized easily. These compounds have high plant branching inhibitory activity, but low activity of promoting the seed germination of root parasitic plants such as plants of the genus *Striga* or *Orobanche,* and as such, can be sprayed over soil. Some of the compounds do not have high plant branching inhibitory activity, but have high activity of promoting the seed germination of root parasitic plants such as plants of the genus *Striga* or *Orobanche,* and as such, can be sprayed over soils in order to induce the suicidal germination of the root parasitic plants.

The compound of the present invention can also be used in root parasitic plant control agents by reducing the viability of root parasitic plants by the inhibition of endogenous strigolactone biosynthesis in host plants through feedback inhibition or by inducing the forced germination of root parasitic plants.

Furthermore, the compound of the present invention has stability in environments and can exhibit its activity over a long period. It is thus expected that strigolactone, which has been difficult to practically apply due to its quantity and stability problems, can be applied practically to agriculture.

In addition, the method for producing the compound of the present invention can synthesize the strigolactone-like compound in a small number of steps using inexpensive materials.

### Brief Description of Drawings

[Figure 1A] Figure 1A shows examples of the structures of the strigolactone-like compounds of the present invention, together with the structure of a strigolactone derivative GR24 known in the art.
[Figure 1B] Figure 1B shows other examples of the structures of the strigolactone-like compounds of the present invention, together with the structure of the strigolactone derivative GR24 known in the art.
[Figure 1C] Figure 1C shows alternative examples of the structures of the strigolactone-like compounds of the present invention.
[Figure 1D] Figure 1D shows alternative examples of the structures of the strigolactone-like compounds of the present invention.
[Figure 1E] Figure 1E shows alternative examples of the structures of the strigolactone-like compounds of the present invention.
[Figure 1F] Figure 1F shows alternative examples of the structures of the strigolactone-like compounds of the present invention.
[Figure 2] Figure 2 is a graph showing the branching inhibitory activity of each strigolactone-like compound against rice.
[Figure 3] Figure 3 is a photograph showing the branching inhibitory activity of each strigolactone-like compound against rice.
[Figure 4] Figure 4 is a graph showing the concentration-dependent branching inhibitory activity of each strigolactone-like compound against rice.
[Figure 5] Figure 5 is a graph showing time-dependent change in the branching inhibitory activity of each strigolactone-like compound against rice.
[Figure 6] Figure 6 is a graph showing the branching inhibitory activity of each strigolactone-like compound against *Arabidopsis thaliana.*
[Figure 7] Figure 7 is a photograph showing the branching inhibitory activity of each strigolactone-like compound against *Arabidopsis thaliana.*
[Figure 8] Figure 8 is a graph showing the germination rate (%) of *Orobanche minor* treated with each strigolactone-like compound.
[Figure 9] Figure 9 is a graph showing the germination rate (%) of *Striga hermonthica* treated with each strigolactone-like compound.
[Figure 10] Figure 10 is a graph showing the amount of endogenous strigolactone exudates from the root of rice treated with each strigolactone-like compound.
[Figure 11] Figure 11 is a graph showing the germination rate (%) of *Striga hermonthica* treated with each strigolactone-like compound.
[Figure 12-1] Figure 12-1 is a graph showing the branching inhibitory activity of each strigolactone-like compound against rice.
[Figure 12-2] Figure 12-2 is a graph showing the branching inhibitory activity of each strigolactone-like compound against rice.
[Figure 12-3] Figure 12-3 is a graph showing the branching inhibitory activity of each strigolactone-like compound against rice.
[Figure 13] Figure 13 is a graph showing the germination rate (%) of *Striga hermonthica* treated with each strigolactone-like compound.
[Figure 14] Figure 14 is a graph showing the branching inhibitory activity of each strigolactone-like compound against rice.
[Figure 15-1] Figure 15-1 is a graph showing the germination rate (%) of *Striga hermonthica* treated with each strigolactone-like compound.
[Figure 15-2] Figure 15-2 is a graph showing the germination rate (%) of *Striga hermonthica* treated with each strigolactone-like compound.
[Figure 15-3] Figure 15-3 is a graph showing the germination rate (%) of *Striga hermonthica* treated with each strigolactone-like compound.
[Figure 16-1] Figure 16-1 is a graph showing the branching inhibitory activity of each strigolactone-like compound against rice.
[Figure 16-2] Figure 16-2 is a graph showing the branching inhibitory activity of each strigolactone-like compound against rice.

### Description of Embodiments

Hereinafter, the present invention will be described in detail. The present application claims the priority based on Japanese Application No. 2010-221766 filed on September 30, 2010 and encompasses the contents described in the specification and/or drawings of the patent application.

The present invention relates to a compound having a strigolactone-like activity (in the present specification, also referred to as a "strigolactone-like compound") and use thereof. In the present invention, the "strigolactone-like activity" means an activity found in natural strigolactone and its derivatives and includes, for example, plant branching inhibitory activity and, in some cases, root parasitic plant germination-promoting activity. In this context, the "inhibition of plant branching" refers to the inhibition of plant branching in the process of plant growth. In the present specification, the "branching" refers to the development and outgrowth of a lateral bud or an auxiliary bud serving as an apex from which a new shoot grows from a stem, a trunk, or a branch. In the present specification, the branching also includes "tillers", which are new lateral buds developed and grown from a part near the root, as shown in plants typified by monocots, etc. The "promotion of root parasitic plant germination" refers to the acceleration of the onset of germination or the enhancement of a germination rate in the process of germination of a root parasitic plant, which grows by parasitizing host plants and absorbing nutrients therefrom.

The strigolactone-like compound of the present invention is represented by the following general formula (III):

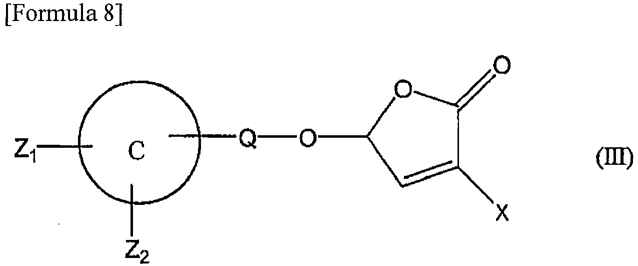

wherein
X represents a lower alkyl group, a lower alkenyl group, a lower alkynyl group, a halogen atom, an OH group, a CN group, a NO₂ group, a CF₃ group, a carboxyl group, or a carboxyl group substituted by a lower alkyl group or a halogen atom;
Q represents a single bond, -CO-, or -CH₂-; and
ring C represents a 6-membered ring represented by the following formula (IVa) or (IVb) or a 5-membered ring represented by the following formula (IVc) or (IVd):

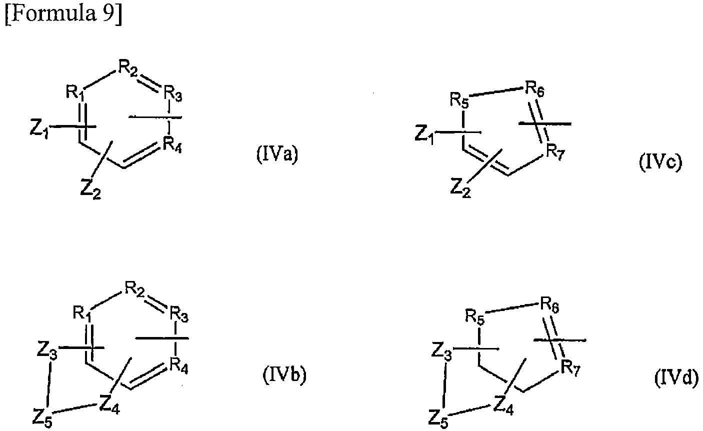

wherein R1, R2, R3, and R4 in the formulas (IVa) and (IVb) each independently represent a carbon atom optionally bonded to a halogen atom or an oxygen atom, a nitrogen atom, a sulfur atom, or an oxygen atom,
R5, R6, and R7 in the formulas (IVc) and (IVd) each independently represent a carbon atom optionally bonded to a halogen atom or an oxygen atom, a nitrogen atom, a sulfur atom, or an oxygen atom,
Z1 and Z2 in the formulas (IVa) and (IVc) are bonded to the ring atoms of ring C and each independently represent a hydrogen atom, a halogen atom, a NO₂ group, a CF₃ group, a CN group, an alkyl group, an alkoxy group, a carboxyl group, a carboxyl group substituted by a lower alkyl group or a halogen atom, an aryl group, an aryloxy group, or an aryl group or an aryloxy group substituted by lower alkyl or a halogen atom,
Z3 and Z4 in the formulas (IVb) and (IVd) each independently represent a carbon atom, a nitrogen atom, a sulfur atom, or an oxygen atom bonded to Z5 to form a 5-membered ring or a 6-membered ring, and Z5 is bonded to Z3 and Z4 to form the 5-membered ring or the 6-membered ring and represents a group constituted of atoms selected from the group consisting of a carbon atom, a nitrogen atom, a sulfur atom, and an oxygen atom.
In the present specification, the "halogen atom" refers to an atom selected from the group consisting of fluorine (F), chlorine (Cl), bromine (Br), and iodine (I).

The "alkyl group" refers to a linear or branched alkyl group having 1 to 10 carbon atoms, preferably 1 to 3 carbon atoms. The alkyl group specifically includes a methyl group, an ethyl group, a n-propyl group, an isopropyl group, a butyl group, an isobutyl group, a t-butyl group, a pentyl group, a hexyl group, a heptyl group, an octyl group, a nonyl group, a decyl group, and the like.

The "lower alkyl group" refers to a linear or branched alkyl group having 1 to 5 carbon atoms, preferably 1 to 3 carbon atoms. The lower alkyl group specifically includes a methyl group, an ethyl group, a n-propyl group, an isopropyl group, a butyl group, an isobutyl group, and a t-butyl group.

The "lower alkenyl group" refers to a linear or branched alkenyl group having 2 to 5 carbon atoms, preferably 2 or 3 carbon atoms. The lower alkenyl group specifically includes an ethenyl group, a propenyl group, and their isomers, and the like.

The "lower alkynyl group" refers to a linear or branched alkynyl group having 2 to 5 carbon atoms, preferably 2 or 3 carbon atoms. The lower alkynyl group specifically includes an ethynyl group and a propynyl group.

The "alkoxy group" refers to a group (R-O-) in which a linear or branched alkyl group having 1 to 5 carbon atoms, preferably 1 to 3 carbon atoms, is bonded to an oxygen atom. The alkoxy group specifically includes a methoxy group, an ethoxy group, and the like.

The "aryl group" refers to an aromatic hydrocarbon group and specifically includes an aryl group, a benzyl group, a tolyl group, a naphthyl group, and the like.

The "aryloxy group" refers to a group in which aromatic hydrocarbon is bonded to an oxygen atom. The aryloxy group specifically includes a phenoxy group, a naphthoxy group, and the like.

The "carboxyl group" is also referred to as a COOR group wherein R can be a hydrogen atom, a lower alkyl group, or a halogen atom.

Each of the groups and rings (including 5- and 6-membered rings) described above may or may not be further substituted, if possible (e.g., at its carbon atom or ring atom), by a halogen atom, an oxygen atom, a lower alkyl group, a lower alkenyl group, a lower alkynyl group, an OH group, a CN group, a NO₂ group, a CF₃ group, a carboxyl group, or the like.

In the general formula (III), Q is preferably a single bond. Specifically, the general formula (III) is preferably the following general formula (IIIa), (IIIb), (IIIc), or (IIId):

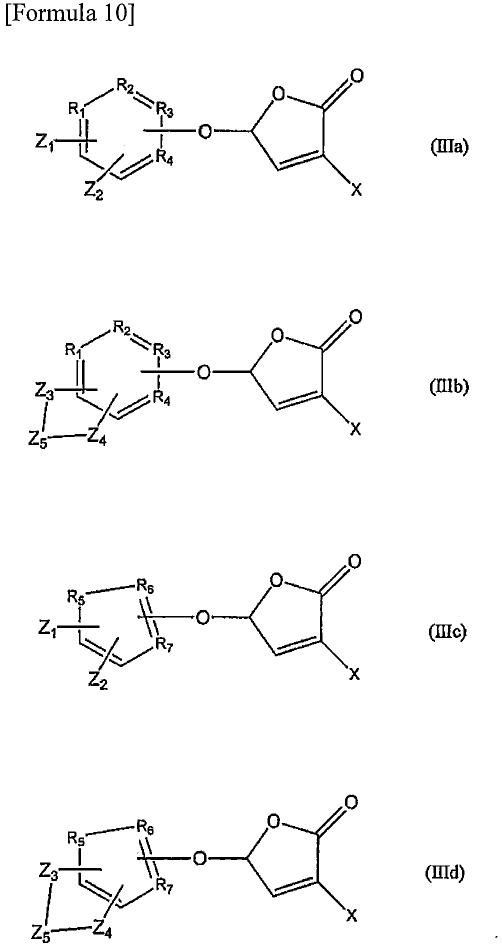

In the general formula (III), each of R1, R2, R3, and R4 in the formula (IVa) or (IVb) is preferably a carbon atom or a carbon atom bonded to a halogen atom or an oxygen atom. Also, each of R5, R6, and R7 in the formula (IVc) or (IVd) is preferably a carbon atom or a carbon atom bonded to a halogen atom or an oxygen atom.

Ring C is preferably a 6-membered ring represented by the formula (IVa) or (IVb), more preferably a 6-membered ring represented by the formula (IVa).

When ring C is a ring represented by the formula (IVa) or (IVc), preferably, Z1 is a hydrogen atom, a halogen atom, a NO₂ group, a CF₃ group, a methoxy group, a COOCH₃ group, a t-butyl group, or a CN group, and Z2 is a hydrogen atom. In this case, Z1 can be located at any position and may be located at an ortho-, meta-, or para-position.

Ring C is preferably an aryl group, an aryloxy group, or an aryl group or an aryloxy group substituted by lower alkyl or a halogen atom.

Alternatively, when ring C is a ring represented by the formula (IVa) or (IVc), preferably, Z1 and Z2 each independently represent a halogen atom, a CN group, a CF₃ group, or a NO₂ group. For example, Z1 and Z2 can be Cl and Cl, Br and Br, F and F, I and I, CF₃ and CF₃, CN and CN, Br and F, Br and CN, Br and NO₂, Br and Cl, or Cl and F in combination. Z1 and Z2 can be located at any position.

In the general formula (III), X is preferably a methyl group.

The preferable structure of the general formula (III) is represented by the following general formula (V):

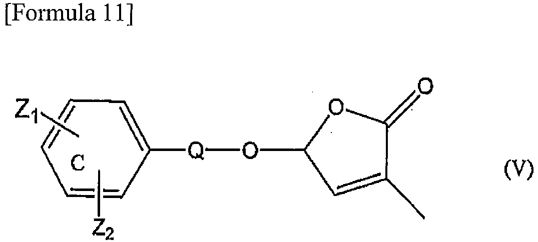

wherein Q represents a single bond or -CO-; and Z1 and Z2 are bonded to the carbon atoms of ring C and each independently represent a hydrogen atom, a halogen atom, a NO₂ group, a CF₃ group, a CN group, an alkyl group, an alkoxy group, a carboxyl group, or a carboxyl group substituted by a lower alkyl group or a halogen atom, or Z1 and Z2 form, together with ring C, an aryl group, an aryloxy group, or an aryl group or an aryloxy group substituted by lower alkyl or a halogen atom.

According to one embodiment, one of Z1 and Z2 in the general formula (V) is a hydrogen atom. According to another embodiment, neither Z1 nor Z2 is a hydrogen atom.

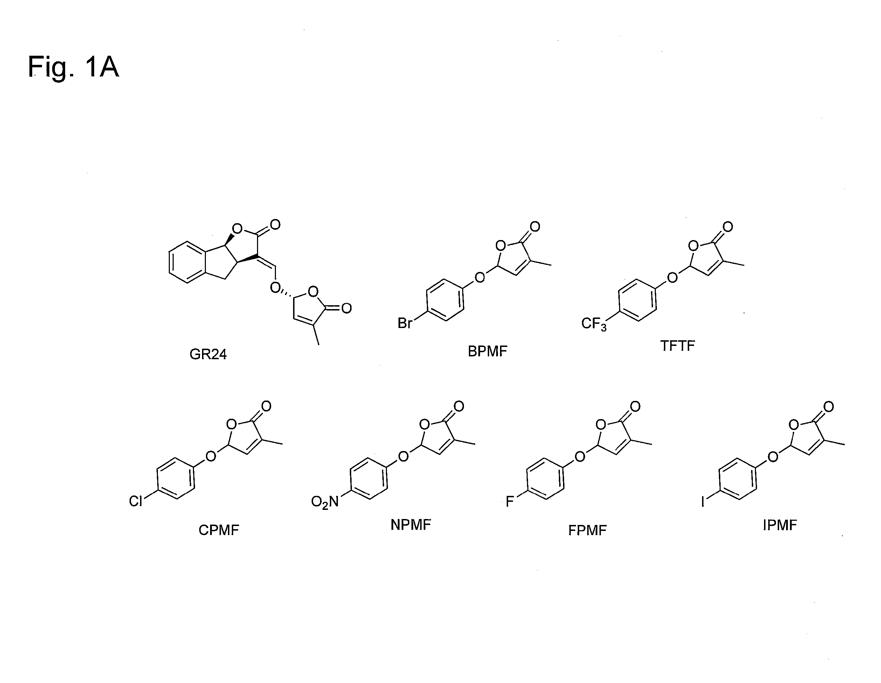

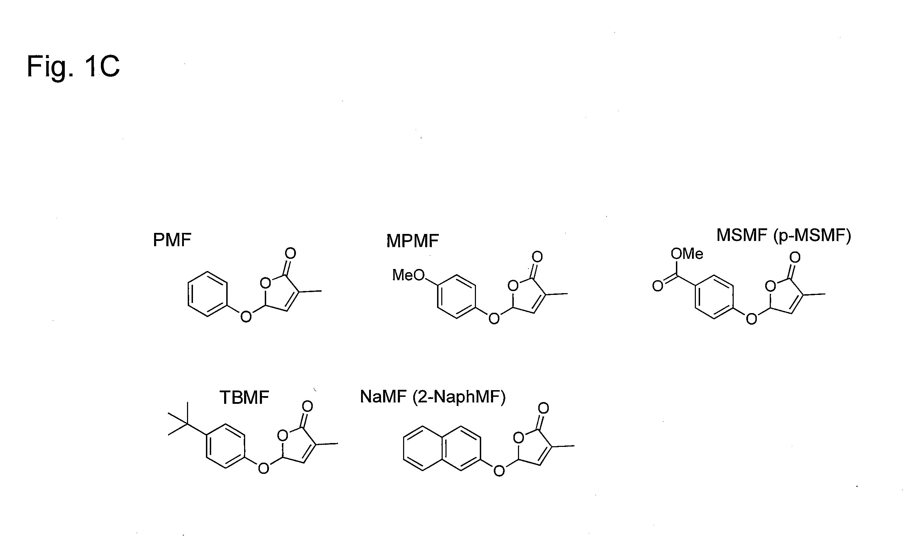

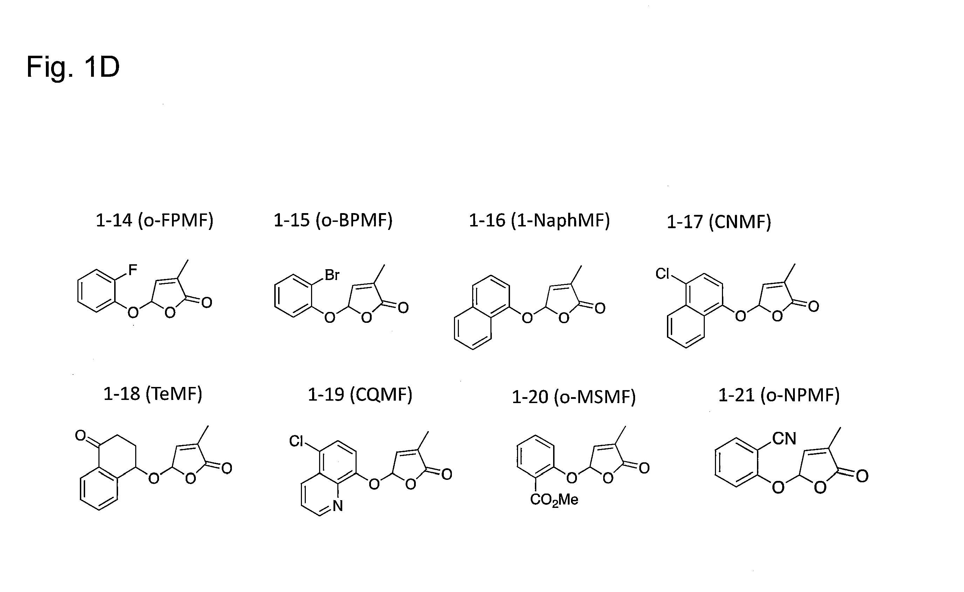

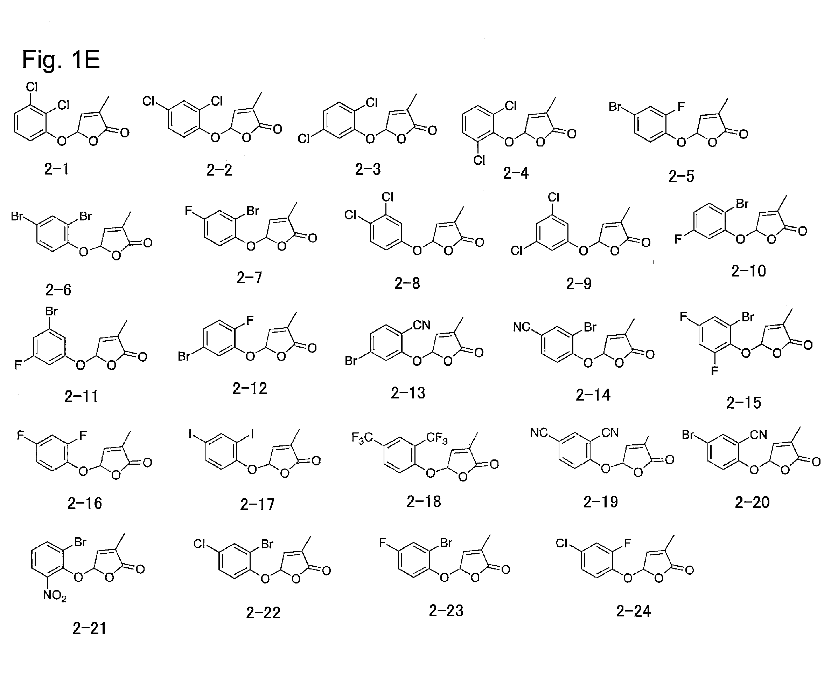

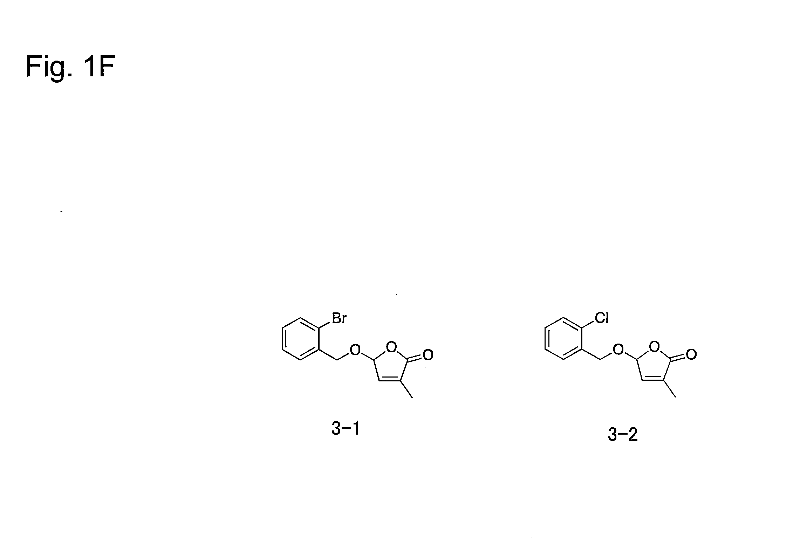

Particularly preferable examples of the compound represented by the general formula (III) include BPMF (p-BPMF and o-BPMF), TFTF, CPMF (p-CPMF, m-CPMF, and o-CPMF), NPMF (p-NPMF and o-NPMF), FPMF (p-FPMF and o-FPMF), IPMF, CBMF (p-CBMF and o-CBMF), PMF, MPMF, MSMF (p-MSMF and o-MSMF), TBMF, NaMF (1-NaphMF and 2-NaphMF), CNMF, TeMF, CQMF, DCMF (2,3-DCMF, 2,4-DCMF, 2,5-DCMF, and 2,6-DCMF), 2-5, 2-6, 2-7, 2-8, 2-9, 2-10, 2-11, 2-12, 2-13, 2-14, 2-15, 2-16, 2-17, 2-18, 2-19, 2-20, 2-21, 2-22, 2-23, 2-24, and o-BBMF shown in Figures 1A to 1F.

The compound represented by the general formula (III) or a salt thereof can be synthesized by a method known in the art. For example, as shown below in the scheme, a ring C derivative represented by the general formula (I) having a dissociable OH group can be reacted with a brominated lactone represented by the general formula (II) under basic conditions to easily obtain the compound represented by the formula (III) at high yields.

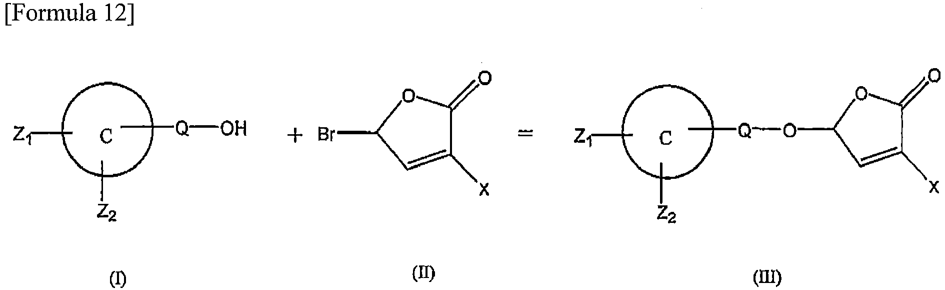

wherein X, Q, ring C, Z1, and Z2 are as defined above.

In general, the ring C derivative represented by the general formula (I) can be obtained easily from a commercially available reagent or can be synthesized by simple functional group transformation. The brominated lactone represented by the general formula (II) may be a commercially available product or can be synthesized conveniently from a lactone derivative. The molar ratio between the ring C derivative and the brominated lactone is not limited and is usually 0.1 to 10, preferably 0.5 to 2.

The reaction is performed under basic conditions using an appropriate solvent. The solvent used is not particularly limited, and examples of the solvent that can be used include organic solvents known in the art, for example, ketones (acetone, methyl ethyl ketone, etc.), aromatic hydrocarbons (benzene, toluene, xylene, etc.), aliphatic hydrocarbons (hexane, heptane, etc.), ethers (diethyl ether, tetrahydrofuran, etc.), halogenated hydrocarbons (methylene dichloride, chloroform, chlorobenzene, dichlorobenzene, etc.), nitriles (acetonitrile, etc.), and aprotic polar solvents (dimethylformamide, dimethylacetamide, etc.); aqueous solvents, for example, water; and mixtures thereof. The basic conditions are adjusted, for example, by dissolving a base in the solvent. Examples of the base that can be used include: inorganic bases, for example, carbonates of alkali metals or alkaline earth metals (potassium carbonate, sodium carbonate, etc.), hydroxides of alkali metals or alkaline earth metals (sodium hydroxide, potassium hydroxide, calcium hydroxide, etc.), and hydrides of alkali metals or alkaline earth metals (sodium hydride, potassium hydride, calcium hydride, etc.); and organic bases, for example, triethylamine, pyridine, sodium ethoxide, and potassium t-butoxide.

The reaction temperature is room temperature, i.e., 5 to 80°C, preferably 10 to 60°C, more preferably 15 to 50°C. The reaction time is 1 to 24 hours, preferably 1 to 10 hours, more preferably 2 to 5 hours. Other reaction conditions preferably involve performing the reaction with stirring.

The compound of interest is obtained by the step. The presence of the compound represented by the formula (III) can be confirmed by an assay technique known in the art, for example, mass spectrometry including NMR and mass spectrum (e.g., EIMS). After the reaction, the compound of interest can be isolated and purified using, alone or in appropriate combination, isolation and purification methods known in the art, for example, filtration, high-performance liquid chromatography (HPLC), column chromatography, ion-exchange chromatography, and recrystallization, though the methods are not limited thereto.

Since the strigolactone-like compound of the present invention has a structure relatively simpler than those of the strigolactone and its derivatives known in the art, the production method described above has the advantages that: the chemical synthesis can be performed easily at low cost; and the compound can be produced at a large scale and can also be practically applied easily.

An acidic group in a substituent in the molecule of the compound represented by the formula (III) can form a base salt with an inorganic or organic base or the like, while a basic group in a substituent in the molecule thereof can form an acid-addition salt with an inorganic or organic acid or the like. Such a base salt and an acid-addition salt are encompassed by the present invention as long as these salts are agriculturally acceptable. Examples of the inorganic acid-addition salt include salts with hydrochloric acid, hydrobromic acid, hydroiodic acid, sulfuric acid, nitric acid, phosphoric acid, and perchloric acid. Examples of the organic acid-addition salt include salts with formic acid, acetic acid, propionic acid, oxalic acid, succinic acid, benzoic acid, p-toluenesulfonic acid, methanesulfonic acid, and trifluoroacetic acid. Examples of the inorganic base salt include salts with alkali metals (sodium, potassium, etc.), alkaline earth metals (calcium, magnesium, etc.), and ammonia. Examples of the organic base salt include salts with dimethylamine, triethylamine, dimethylaniline, piperazine, pyrrolidine, piperidine, pyridine, phenylethylamine, benzylamine, ethanolamine, and diethanolamine. These salts are known in the art and can be formed by those skilled in the art according to a routine method.

Also, the compound represented by the formula (III) may encompass stereoisomers such as optical isomers and geometric isomers based on asymmetric carbon atoms and double bonds. Such isomers and their mixtures are also encompassed in the scope of the present invention. In addition, the compound represented by the formula (III) encompasses all of compounds in the forms of solvates (e.g., hydrates), in crystalline and/or non-crystalline forms, and in the forms of prodrugs.

The strigolactone-like compound of the present invention represented by the formula (III) has an activity equal to or higher than that of natural strigolactone, is stable in environments, and can be chemically synthesized by a convenient step and at low cost. The strigolactone mainly has two activities, one of which is plant branching inhibitory activity, and the other of which is root parasitic plant germination-promoting activity. The strigolactone-like compound of the present invention includes those having no or weak seed germination-promoting effect on root parasitic plants, and those having root parasitic plant germination-promoting activity. Thus, these compounds can be used separately according to the presence or absence of the activity.

The strigolactone-like compound of the present invention exhibits a strigolactone-like activity against plants and as such, can be used in an agricultural chemical composition. Specifically, the present invention provides an agricultural chemical composition comprising at least one strigolactone-like compound described above and/or salt(s) thereof and an agriculturally acceptable carrier.

The agricultural chemical composition of the present invention has plant branching inhibitory activity and as such, can be used as a plant branching inhibitor. The "plant branching inhibitor" refers to a drug having a plant branching inhibitory effect. Plant branching can be regulated using the agricultural chemical composition of the present invention. The plant branching inhibitor is useful, for example, in decreasing the number of branches by the inhibition of branching and relatively promoting apical bud outgrowth. For example, the plant branching inhibitor can be applied to tobacco to inhibit lateral buds or can be applied to a horticultural plant such as chrysanthemum to save time and effort required for disbudding. On the contrary, the plant branching inhibitor can also be applied as an agricultural chemical to fallow soil or the like to prevent the proliferation or exuberance of weeds by the inhibition of their tillers.

The plant targeted by the plant branching inhibitor of the present invention may be any of monocots and dicots as long as the branching of the plant can be inhibited by the strigolactone-like compound of the present invention. The target plant is preferably a crop plant. Examples of the crop plant include, but not limited to: cereal plants such as rice, wheat, barley, and corn; oil plants such as rape, sunflower, and sesame; vegetable plants, for example, plants of the family *Solanaceae* (tomato, bell pepper, eggplant, potato, chili pepper, tobacco, etc.), plants of the family *Cucurbitaceae* (melon, cucumber, pumpkin, etc.), plants of the family *Convolvulaceae* (sweetpotato, etc.), plants of the family *Umbelliferae* (carrot, cicely, celery, etc.), plants of the family *Compositae* (burdock, garland chrysanthemum, etc.), plants of the family *Chenopodiaceae* (spinach, chard, etc.), plants of the family *Alliaceae* (leek, scallion, garlic chive, Chinese onion, etc.), and plants of the family *Zingiberaceae* (ginger, etc.); fruit plants such as grape, apple, and mandarin orange; and plants of the family *Leguminosae* such as soybean, peanut, and pea. Alternatively, the target plant may be a horticultural plant (rose, carnation, chrysanthemum, etc.) or any of other plants (cotton, linum, etc.).

In the case of having root parasitic plant germination-promoting activity, the agricultural chemical composition of the present invention can reduce or control root parasitic plants by inducing the forced germination of the root parasitic plants. In the case of having no root parasitic plant germination-promoting activity, the agricultural chemical composition of the present invention can reduce or control root parasitic plants by reducing the viability of the root parasitic plants by the inhibition of endogenous strigolactone biosynthesis in host plants through feedback inhibition. Thus, the agricultural chemical composition of the present invention can be used as a root parasitic plant control agent. The "root parasitic plant control agent" refers to a drug having the effect of inhibiting and/or reducing the germination and/or growth of root parasitic plants, which grow by parasitizing host plants and absorbing nutrients therefrom. For example, the agricultural chemical composition of the present invention can be applied to a field before cultivation of crop plants or horticultural plants to forcedly promote the germination of root parasitic plants that parasitize the host crop plants or horticultural plants. As a result, the root parasitic plants can be controlled easily. Alternatively, the agricultural chemical composition of the present invention can be applied to host plants that are parasitized by root parasitic plants to decrease the amount of strigolactone in the host plants through the feedback inhibition of endogenous strigolactone biosynthesis by the strigolactone-like compound of the present invention. Accordingly, the root parasitic plants become deficient in strigolactone and can no longer parasitize the host plants. As a result, damage caused by the root parasitic plants to the host plants can be reduced.

The target root parasitic plant to be controlled by the root parasitic plant control agent of the present invention is not particularly limited as long as the root parasitic plant can be controlled by the strigolactone-like compound of the present invention. The root parasitic plant is, for example, a plant belonging to the family *Orobanchaceae.* More specifically, a plant belonging to the genus *Orobanche,* for example, *Orobanche minor,* or a plant belonging to the genus *Striga,* for example, *Striga hermonthica* can be controlled by the root parasitic plant control agent of the present invention.

The agricultural chemical composition of the present invention is provided in a form comprising at least one strigolactone-like compound of the present invention or salt thereof as an active ingredient combined with an agriculturally acceptable carrier appropriate for intended use. The "agriculturally acceptable carrier" refers to a substance that facilitates the application of the agricultural chemical composition and inhibits or prevents its decomposition or/and regulates the rate at which the agricultural chemical composition acts. This substance has no or a little harmful effect on environments such as soil and water quality or on animals, particularly, humans, even when applied to the cultivation of plants including outdoor and indoor plants. For example, a carrier and an adjuvant can be used.

Any of liquid carriers and solid carriers can be used as the carrier. Preferable examples of the liquid carriers (vehicles) include, water, alcohols (methyl alcohol, ethyl alcohol, n-propyl alcohol, isopropyl alcohol, ethylene glycol, etc.), ethers (dioxane, tetrahydrofuran, ethylene glycol monomethyl ether, diethylene glycol monomethyl ether, propylene glycol monomethyl ether, etc.), ketones (acetone, methyl ethyl ketone, etc.), aliphatic hydrocarbons (kerosine, kerosene, fuel oil, machine oil, etc.), aromatic hydrocarbons (benzene, toluene, xylene, solvent naphtha, methylnaphthalene, etc.), halogenated hydrocarbons (methylene chloride, chloroform, carbon tetrachloride, etc.), amides (dimethylformamide, dimethylacetamide, etc.), esters (acetic acid ethyl ester, acetic acid butyl ester, fatty acid glycerin ester, etc.), nitriles (acetonitrile, propionitrile, etc.), and mixed solvents of two or more of them.

Preferable examples of the solid carriers include plant-derived powders (e.g., soybean flour, tobacco powder, wheat flour, and wood flour), mineral-derived powders (e.g., kaolin, bentonite, clay, talc such as talcum powder or phrophyllite, and silica such as diatomaceous earth or mica powder), alumina, sulfur dust, activated carbon, calcium carbonate, ammonium sulfate, sodium bicarbonate, lactose, urea, and mixtures of two or more of them.

Preferable examples of the adjuvant include natural mineral powders, synthetic mineral powders, emulsifiers, dispersants, suspending agents, surfactants, antioxidants, antiseptics, spreading agents, wetting agents, penetrants, mucilages, stabilizers, fixing agents, and adsorbents.

Examples of the natural mineral powders include kaolin, clay, talc, and chalk. Examples of the synthetic mineral powders include highly dispersible silica and silicate.

Examples of the emulsifiers include nonionic emulsifiers and anionic emulsifiers (e.g., polyoxyethylene fatty alcohol ether, alkyl sulfonate, and aryl sulfonate). Examples of the dispersants include lignosulfite waste liquor and methylcellulose.

The surfactants may be any of cationic surfactants, anionic surfactants, and nonionic surfactants and may be used alone or as a mixture of two or more types thereof. Examples of the surfactants include lignosulfonic acid, naphthalenesulfonic acid, phenolsulfonic acid, alkali metal salts, alkaline earth metal salts, and ammonium salts of dibutylnaphthalenesulfonic acid, alkylaryl sulfonate, alkyl sulfate, alkyl sulfonate, fatty alcohol sulfate, fatty acid and sulfated fatty alcohol glycol ethers, condensates of sulfonated naphthalene or a naphthalene derivative and formaldehyde, condensates of naphthalene or naphthalenesulfonic acid, phenol, and formaldehyde, polyoxyethylene octyl phenyl ether, ethoxylated isooctylphenol, octylphenol, nonylphenol, alkylphenyl polyglycol ether, tributylphenyl polyglycol ether, tristearylphenyl polyglycol ether, alkylaryl polyether alcohol, condensates of alcohol or fatty alcohol and ethylene oxide, ethoxylated castor oil, polyoxyethylene alkyl ether, ethoxylated polyoxypropylene, lauryl alcohol polyglycol ether acetal, sorbitol ester, lignosulfite waste liquor, and methylcellulose.

Examples of the antioxidants include dibutylhydroxytoluene and 4,4-thiobis-6-tert-butyl-3-methylphenol. Examples of the antiseptics include sorbic acid and potassium sorbate.

The agricultural chemical composition of the present invention may comprise, in addition to the agriculturally acceptable carrier, other active ingredients for agricultural chemicals, for example, other plant branching inhibitors, pesticides, insecticides, herbicides, fungicides, and fertilizers (e.g., urea, ammonium nitrate, and superphosphate), without influencing the functions or effects of the agricultural chemical composition of the present invention.

The content of the active ingredient in the agricultural chemical composition of the present invention differs depending on the type of the active ingredient (strigolactone-like compound), the form of the composition, an application method, intended application, the type of a recipient plant, etc. The content of the active ingredient in the agricultural chemical composition can be determined in consideration of various conditions so that the active ingredient contained in the agricultural chemical composition achieves the desired amount for the target plant after application within the scope of the technical common sense of this field. The content of the active ingredient is generally approximately 0.1 to 99% by weight, preferably approximately 1 to 80% by weight, more preferably approximately 1 to 50% by weight, with respect to the total amount of the agricultural chemical composition. Specifically, usually approximately 1 to 80% by weight, preferably approximately 1 to 20% by weight, is appropriate for use in the form of an emulsion, a solution, a wettable powder (e.g., a water dispersible granule), or the like. Also, usually approximately 0.1 to 50% by weight, preferably approximately 0.1 to 20% by weight, is appropriate for use in the form of an oil solution, a dust, or the like. Alternatively, usually approximately 1 to 50% by weight, preferably approximately 5 to 20% by weight, is appropriate for use in the form of a granule or the like. The content of the ingredients (carrier and adjuvant) other than the active ingredient differs depending on the type and content of the active ingredient, the types and contents of other active ingredients for agricultural chemicals, and the form and application method of the composition, etc., and is usually approximately 0.001 to 99.9% by weight, preferably approximately 1 to 99% by weight. For use in the form of an emulsion, a wettable powder (e.g., a water dispersible granule), or the like, the composition may be diluted or increased (e.g., approximately 100 to 5000 times) appropriately with water or the like for spraying. The amount of such a diluent can also be taken into consideration.

The form of the agricultural chemical composition of the present invention can be any of routine pharmaceutical forms, for example, solutions, oil dispersions, emulsions, microemulsions, flowables, wettable powders, suspensions, dusts, powders, pastes, pellets, microcapsules, ointments, aerosols, tablets, and granules, which can be applied directly by spraying, nebulization, dusting, coating, and/or dipping. These preparations can be prepared according to a routine method by: dissolving or dispersing the active ingredient in a liquid carrier or mixing or adsorbing the active ingredient with or onto an appropriate solid carrier; and, if necessary, adding thereto, for example, an emulsifier, a suspending agent, a spreading agent, a penetrant, a wetting agent, a mucilage, and/or a stabilizer.

The method for applying the agricultural chemical composition of the present invention is not particularly limited as long as its branching inhibitory effect can be imparted to the target plant or the target root parasitic plant can be controlled. The agricultural chemical composition of the present invention can be applied to the whole or a portion (seeds, seedlings, roots, etc.) of the plant or to a medium or soil for growth of the plant by a method known in the art.

For example, for hydroponic culture, the agricultural chemical composition of the present invention can be added into a hydroponic culture solution (medium). This method is preferable because the method can easily and uniformly apply the agricultural chemical composition of the present invention. In this case, the applied agricultural chemical composition of the present invention is absorbed from the root and thereby spread throughout the plant where the active ingredient can then exert the effects of the present invention.

Alternatively, the agricultural chemical composition of the present invention may be applied to soil by spraying, nebulization, dusting, or the like. This method is convenient for application to a wide area such as a field. Of course, the agricultural chemical composition of the present invention can be used in indoor soil cultivation. Alternatively, the agricultural chemical composition of the present invention may be enclosed in a sustained-release inclusion body and applied indirectly. In this case as well, the applied agricultural chemical composition of the present invention is absorbed from the root and thereby spread throughout the plant where the active ingredient can then exert the effects of the present invention.

Furthermore, a drug in a liquid form comprising the agricultural chemical composition of the present invention may be applied to the whole or a portion of the plant by coating, nebulization, dipping, injection, or spraying. A site for the application such as coating is not limited and can be the desired location such as seeds, stems, or the base of the petiole. This method is effective for locally inhibiting branching effects at the particular site of a plant.

Since the agricultural chemical composition of the present invention has a higher activity than that of the strigolactone derivatives known in the art as described above, the agricultural chemical composition applied in a smaller amount can exert large functions and effects. However, the specific amount of the agricultural chemical composition applied varies depending on the type of the active ingredient used, the type of a recipient plant, intended application, and the application method, etc. For example, even the inhibition of the branching of the same target plant requires applying the agricultural chemical composition in a larger amount in the case of application intended to completely inhibit the branching than that in the case of application intended to inhibit the branching to some extent. Also, direct application to a field in expectation of effects equivalent to those in hydroponic culture requires applying the agricultural chemical composition in a larger amount. This is because the active ingredient is generally decomposed at a faster rate in soil by the decomposition action of microbes than that in a hydroponic culture solution. Such an amount can be determined appropriately by those skilled in the art according to the situation, purpose, and need.

The amount of the agricultural chemical composition of the present invention applied is an amount that can achieve the intended effects, for example, approximately 0.01 to 1.5 kg/ha, preferably approximately 1 to 1500 g/ha, of the active ingredient. When the agricultural chemical composition of the present invention is used in the treatment of seeds, the amount of the agricultural chemical composition applied is usually 1 to 1500 g/100 kg seed, preferably 10 to 500 g/100 kg seeds, in terms of the amount of the active ingredient.

As an example of the amount of the agricultural chemical composition applied, the agricultural chemical composition of the present invention can be added at an active ingredient concentration (final concentration) of 0.1 nM to 50 µM, preferably 1 nM to 10 µM, more preferably 10 nM to 1 µM, to a hydroponic culture solution, in order to completely inhibit branching in the hydroponic culture of rice. Usually, the active ingredient concentration of approximately 10 nM can be expected to offer a sufficient inhibitory effect.

The present invention also encompasses a method for inhibiting the branching of a plant and a method for controlling a root parasitic plant, comprising applying the agricultural chemical composition of the present invention to the plant or to a medium or soil for growth of the plant, as described above.

The strigolactone-like compound serving as the active ingredient of the present invention is relatively stable in environments and thus has the advantages that: the compound is applied in a small amount or at a small number of times; and its activity is sustained over a long period. These advantages are excellent in light of the fact that in the case of auxins, which are also plant hormones, natural indoleacetic acid cannot be practically applied due to its instability in environments, whereas the development of the synthetic auxin 2,4-D has led to wide use as a plant hormone-disrupting herbicide.

### Examples

Hereinafter, the present invention will be described specifically with reference to Examples. However, Examples shown herein are provided for illustrative purposes and do not limit the scope of the present invention by any means.

### [Synthesis Example 1] Synthesis of 5-(4-bromophenoxy)-3-methylfuran-2(5H)-one

2 g of potassium carbonate was dissolved in 30 ml of water. To this solution, 100 ml of methylene dichloride containing 1.73 g (10 mmol) of 4-brominated phenol dissolved therein was added, and the mixture was stirred. 1.77 g (10 mmol) of brominated lactone was added thereto, and the mixture was stirred at room temperature for 3 hours. After the completion of reaction, the organic layer was extracted using a separating funnel, and the aqueous layer was further subjected to extraction with methylene dichloride (50 ml) twice. The organic layers were combined, dried over sodium sulfate, and then concentrated. The residue was then purified by silica gel column chromatography (hexane:ethyl acetate = 8:2) and recrystallized from a hexane solution to obtain the compound of interest (Figure 1A, BPMF). Yield: 86%.

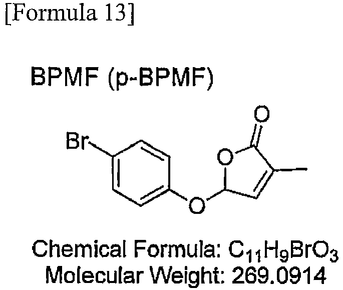

¹H-NMR(CDCl₃, 500 MHz)
d 2.01(s, 3H, CH=CCH₃), 6.24(d, 1H, C=CHCH), 6.98(d, 1H, C=CH),
7.01(d, 2H, ArH), 7.43(d, 2H, ArH).

### [Synthesis Example 2] Synthesis of strigolactone-like compound

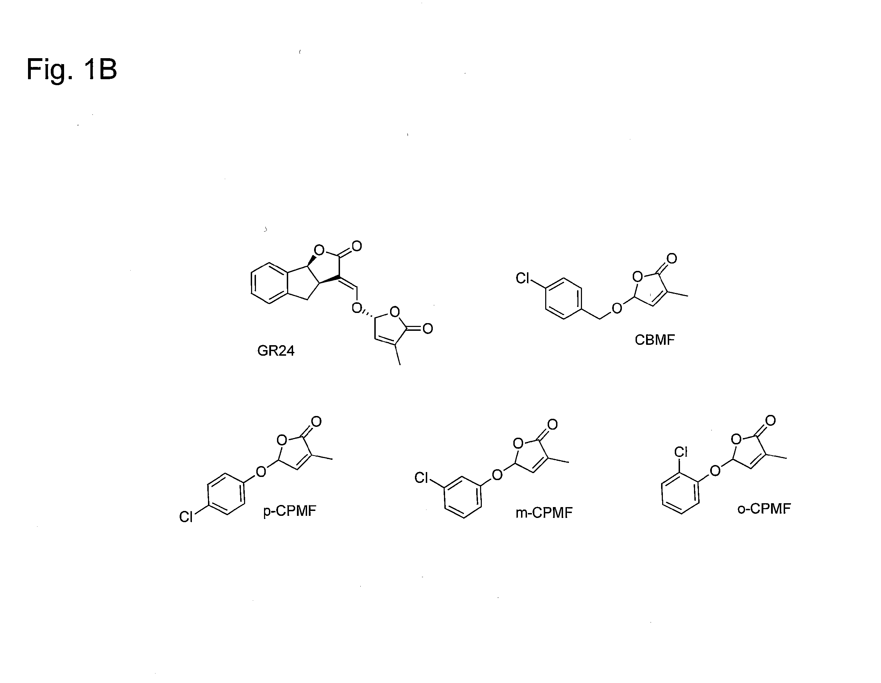

Strigolactone-like compounds TFTF, CPMF, NPMF, FPMF, and IPMF shown in Figure 1A were synthesized in the same way as in Synthesis Example 1. Also, strigolactone-like compounds CBMF, m-CPMF, and o-CPMF shown in Figure 1B were similarly synthesized.

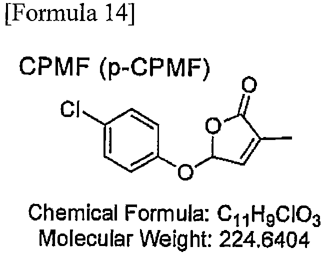

¹H-NMR(CDCl₃, 500 MHz)
d 2.010(s, 3H, CH=CCH₃), 6.230(d, 1H, C=CHCH), 6.966(d, 1H, C=CH), 7.065(d, 2H, ArH), 7.286(d, 2H, ArH).

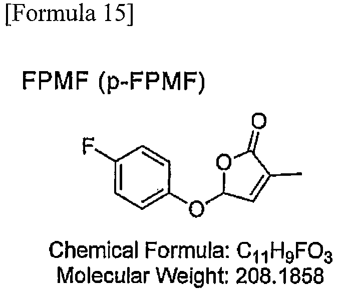

¹H-NMR(CDCl₃, 500 MHz)
d 2.01(s, 3H, CH=CCH₃), 6.22(d, 1H, C=CHCH), 6.99(d, 1H, C=CH), 7.03(d, 2H, ArH), 7.10(d, 2H, ArH).

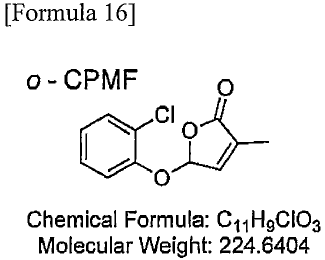

¹H-NMR(CDCl₃, 500 MHz)
d 2.020(s, 3H, CH=CCH₃), 6.253(d, 1H, C=CHCH), 7.080(m, 2H, C=CH, ArH), 7.262(dd, 1H, ArH), 7.343(dd, 1H, ArH), 7.406(dd, 1H, ArH).

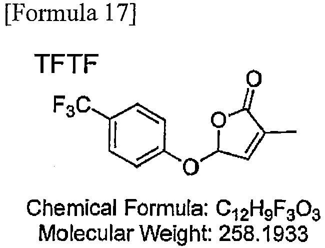

¹H-NMR(CDCl₃, 500 MHz)
d 2.035(s, 3H, CH=CCH₃), 6.335(d, 1H, C=CHCH), 7.007(d, 1H, C=CH), 7.215 (d, 2H, ArH), 7.610(d, 2H, ArH).

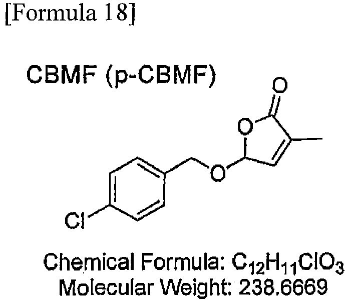

¹H-NMR(CDCl₃, 500 MHz)
d 1.998(s, 3H, CH=CCH₃), 5.846(d, 1H, C=CHCH), 6.538(dt, 2H, ArCH₂O-), 7.118(d, 1H, C=CH), 7.299(d, 2H, ArH), 7.423(d, 2H, ArH).

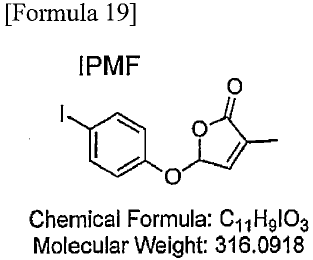

¹H-NMR(CDCl₃, 500 MHz)
d 2.013(s, 3H, CH=CCH₃), 6.238(d, 1H, C=CHCH), 6.905(d, 2H, ArH), 6.971(d, 1H, C=CH), 7.625(d, 2H, ArH).

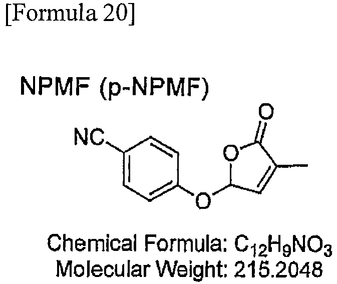

¹H-NMR(CDCl₃, 500 MHz)
d 2.039(s, 3H, CH=CCH₃), 6.341(d, 1H, C=CHCH), 7.011(d, 1H, C=CH),
7.205(d, 2H, ArH), 7.654(d, 2H, ArH).

### [Synthesis Example 3] Synthesis of strigolactone-like compound

Strigolactone-like compounds PMF, MPMF, MSMF (p-MSMF), TBMF, and NaMF (2-NaphMF) shown below were synthesized in the same way as in Synthesis Example 1.

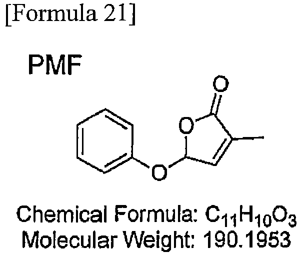

¹H-NMR(CDCl₃, 500 MHz)
d 2.004(s, 3H, CH=CCH₃), 6.294(d, 1H, C=CHCH), 6.983(d, 1H, C=CH), 7.122(m, 3H, ArH), 7.335(t, 2H, ArH).

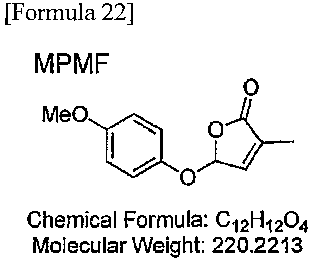

¹H-NMR(CDCl₃, 500 MHz)
d 1.996(s, 3H, CH=CCH₃), 3.788(s, 1H, -O-CH₃), 6.191(d, 1H, C=CHCH), 6.851(d, 2H, ArH), 6.962(d, 1H, C=CH), 7.075(d, 2H, ArH).

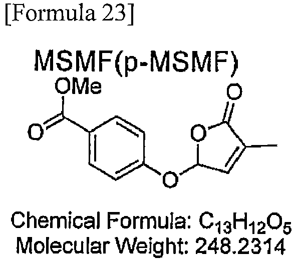

¹H-NMR(CDCl₃, 500 MHz)
d 2.033(s, 3H, CH=CCH₃), 3.907(s, 1H, (CO)OCH₃), 6.360(d, 1H, C=CHCH), 7.006(d, 1H, C=CH), 7.159(d, 2H, ArH), 8.036(d, 2H, ArH).

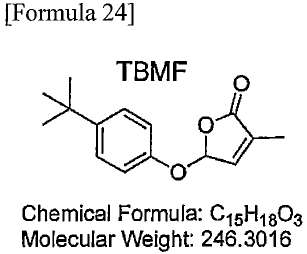

¹H-NMR(CDCl₃, 500 MHz)
d 1.308(s, 9H, C(CH₃)₃), 2.006(s, 3H, CH=CCH₃), 6.272(d, 1H, C=CHCH), 6.972(d, 1H, C=CH),
7.063(d, 2H, ArH), 7.344(d, 2H, ArH).

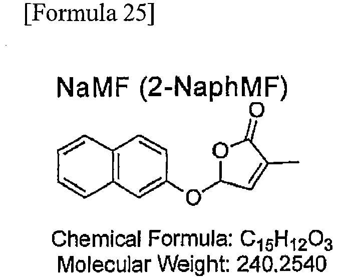

¹H-NMR(CDCl₃, 500 MHz)
d 2.07(s, 3H, CH=CCH₃), 6.47(d, 1H, C=CHCH), 7.12(d, 1H, C=CH),
7.31 (d, 1H, ArH), 7.43 (dd, 1 H, ArH), 7.51(m, 2H, ArH), 7.60(d, 1H, ArH),
7.84(m, 1H, ArH), 8.15(m, 1H, ArH).

### [Synthesis Example 4] Synthesis of strigolactone-like compound

Strigolactone-like compounds shown below were synthesized in the same way as in Synthesis Example 1.

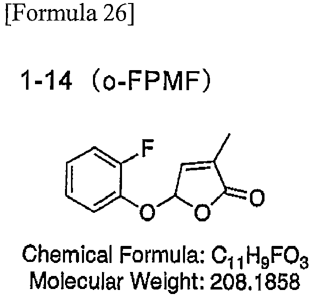

¹H-NMR(CDCl₃, 500 MHz)
d 2.00(s, 3H, CH=CCH₃), 6.27(d, 1H, C=CHCH), 7.04(d, 1H, C=CH), 7.06-7.14(m, 3H, ArH), 7.29-7.33 (m, 1H, ArH).

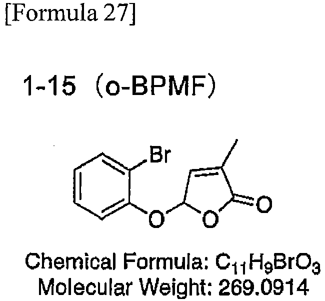

¹H-NMR(CDCl₃, 500 MHz)
d 2.03(s, 3H, CH=CCH₃), 6.24(d, 1H, C=CHCH), 7.00-7.04(m, 1H, ArH), 7.08(d, 1H, C=CH), 7.29-7.35(m, 2H, ArH), 7.58 (dd, 1H, ArH).

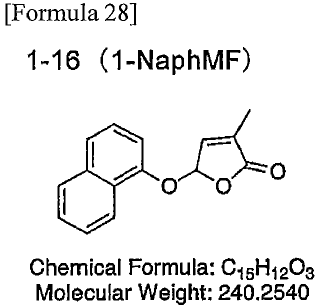

¹H-NMR(CDCl₃, 500 MHz)
d 2.05(s, 3H, CH=CCH₃), 6.47(d, 1H, C=CHCH), 7.12(d, 1H, C=CH), 7.31(d, 1H, ArH), 7.42(t, 1H, ArH), 7.47-7.53(m, 2H, ArH), 7.60(d, 1H, ArH), 7.84(dd, 1H, ArH), 8.14(dd, 1H, ArH).

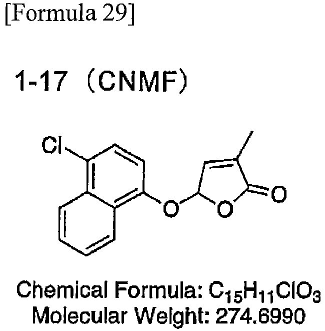

¹H-NMR(CDCl₃, 500 MHz)
d 2.07(s, 3H, CH=CCH₃), 6.44(d, 1H, C=CHCH), 7.12(d, 1H, C=CH),
7.23(m, 1H, ArH), 7.48-7.66(m, 3H, ArH), 8.17(dd, 1H, ArH), 8.25(dd, 1H, ArH).

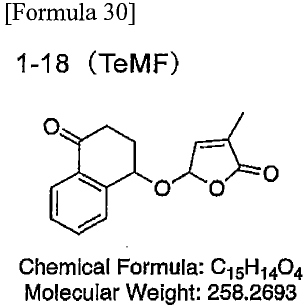

¹H-NMR(CDCl₃, 500 MHz)
d 2.02(s, 3H, CH=CCH₃), 2.08-2.19(m, 1H, ArCH), 2.61-2.68(m, 2H, COCH₂CH₂), 2.87-2.94(m, 2H, COCH₂), 6.30(d, 1H, C=CHCH), 7.02(d, 1H, C=CH), 7.22-7.37(m, 2H, ArH), 7.43(dd, 1H, ArH), 7.83(dd, 1H, ArH).

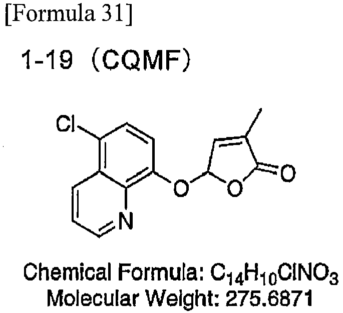

¹H-NMR(CDCl₃, 500 MHz)
d 2.03(s, 3H, CH=CCH₃), 6.62(d, 1H, C=CHCH), 7.27(d, 1H, C=CH),
7.50(m, 1H, ArH), 7.54-7.63(m, 2H, ArH), 8.59(dd, 1H, ArH), 9.01(d, 1H, ArH).

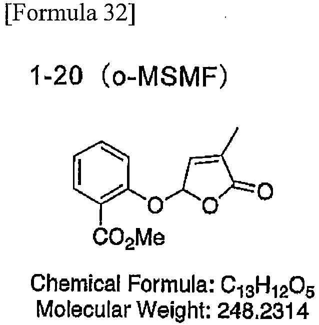

¹H-NMR(CDCl₃, 500 MHz)
d 2.01(s, 3H, CH=CCH₃), 3.90(s, 3H, ArOCH₃), 6.25(s, 1H, C=CHCH), 7.11(s, 1H, C=CH), 7.22(t, 1H, ArH), 7.38(d, 1H, ArH), 7.53(t, 1H, ArH), 7.87(d, 1H, ArH).

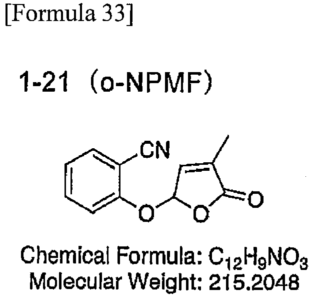

¹H-NMR(CDCl₃, 500 MHz)
d 2.04(s, 3H, CH=CCH₃), 6.35(t, 1H, C=CHCH), 7.10(s, 1H, C=CH),
7.20(dt, 1H, ArH), 7.40(d, 1H, ArH), 7.59-7.64(m, 2H, ArH).

### [Synthesis Example 5] Synthesis of strigolactone-like compound

Strigolactone-like compounds shown below were synthesized in the same way as in Synthesis Example 1.

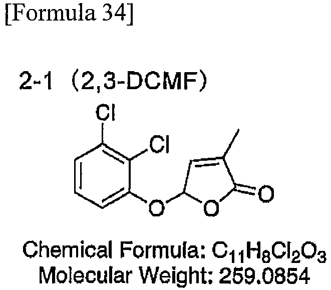

¹H-NMR(CDCl₃, 500 MHz)
d 2.03(s, 3H, CH=CCH₃), 6.24(d, 1H, C=CHCH), 7.07(d, 1H, C=CHCH), 7.20(t, 1H, ArH), 7.25-7.28(m, 2H, ArH).

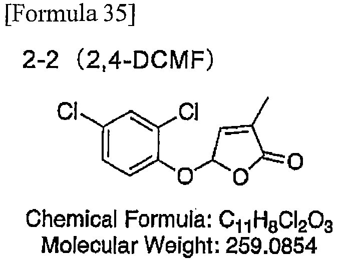

¹H-NMR(CDCl₃, 500 MHz)
d 2.02(s, 3H, CH=CCH₃), 6.20(d, 1H, C=CHCH), 7.05(d, 1H, C=CHCH), 7.22-7.29(m, 2H, ArH), 7.41 (d, 1H, ArH).

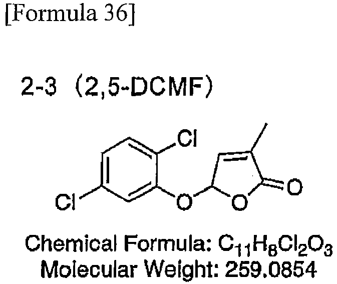

¹H-NMR(CDCl₃, 500 MHz)
d 2.03(s, 3H, CH=CCH₃), 6.24(d, 1H, C=CHCH), 7.06-7.08(m, 2H, C=CHCH, ArH), 7.33(d, 1H, ArH), 7.37 (d, 1H, ArH).

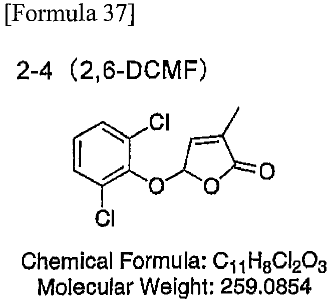

¹H-NMR(CDCl₃, 500 MHz)
d 2.03(s, 3H, CH=CCH₃), 6.30(d, 1H, C=CHCH), 7.10(t, 1H, ArH), 7.14(d, 1H, C=CHCH), 7.35 (d, 2H, ArH).

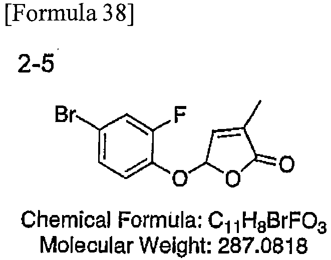

¹H-NMR(CDCl₃, 500 MHz)
d 2.00(s, 3H, CH=CCH₃), 6.23(d, 1H, C=CHCH), 7.03(d, 1H, C=CHCH), 7.18-7.31 (m, 3H, ArH).

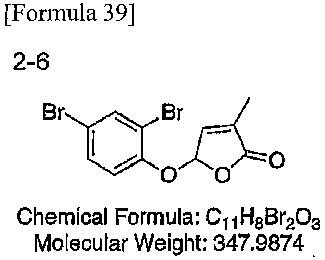

¹H-NMR(CDCl₃, 500 MHz)
d 2.03(s, 3H, CH=CCH₃), 6.12(d, 1H, C=CHCH), 7.06(d, 1H, C=CHCH), 7.21(d, 1H, ArH), 7.43(dd, 1H, ArH), 7.72 (d, 1H, ArH).

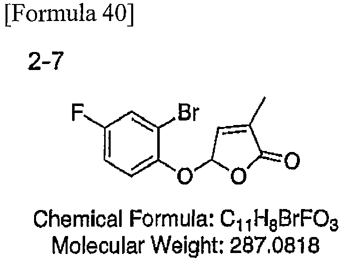

¹H-NMR(CDCl₃, 500 MHz)
d 2.02(s, 3H, CH=CCH₃), 6.16(d, 1H, C=CHCH), 7.03(m, 1H, ArH), 7.07(d, 1H, C=CHCH), 7.29-7.34(m, 2H, ArH).

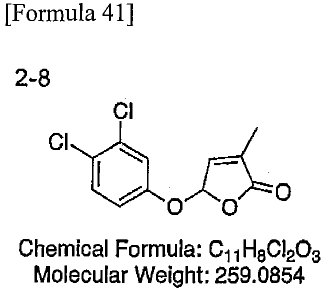

¹H-NMR(CDCl₃, 500 MHz)
d 2.02(s, 3H, CH=CCH₃), 6.23(d, 1H, C=CHCH), 6.97-7.01(m, 2H, C=CHCH, ArH), 7.27(d, 1H, ArH), 7.40 (d, 1H, ArH).

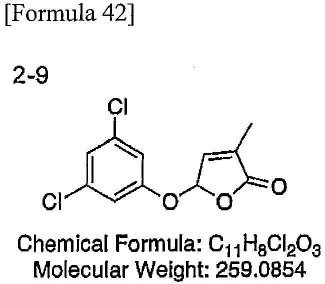

¹H-NMR(CDCl₃, 500 MHz)
d 2.03(s, 3H, CH=CCH₃), 6.24(d, 1H, C=CHCH), 6.97(d, 1H, C=CHCH), 7.06(d, 2H, ArH), 7.12 (t, 1H, ArH).

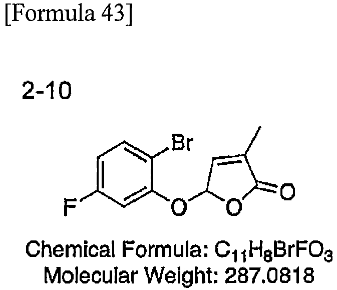

¹H-NMR(CDCl₃, 500 MHz)
d 2.04(s, 3H, CH=CCH₃), 6.23(d, 1H, C=CHCH), 6.77(dt, 1H, ArH), 7.07(d, 1H, C=CHCH), 7.11 (dd, 1H, ArH) 7.52(dd, 1H, ArH).

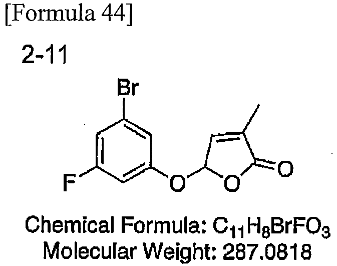

¹H-NMR(CDCl₃, 500 MHz)
d 2.03(s, 3H, CH=CCH₃), 6.24(d, 1H, C=CHCH), 6.82(td, 1H, ArH), 6.97(d, 1H, C=CHCH), 7.02 (dd, 1H, ArH) 7.12(d, 1H, ArH).

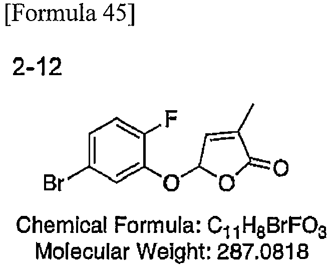

¹H-NMR(CDCl₃, 500 MHz)
d 2.02(s, 3H, CH=CCH₃), 6.25(d, 1H, C=CHCH), 6.99-7.04(m, 2H, C=CHCH, ArH), 7.21 (ddd, 1H, ArH) 7.48(dd, 1H, ArH).

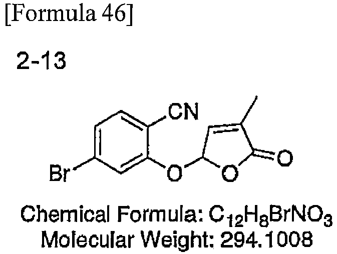

¹H-NMR(CDCl₃, 500 MHz)
d 2.05(s, 3H, CH=CCH₃), 6.32(s, 1H, C=CHCH), 7.09(s, 1H, C=CHCH), 7.35(dd, 1H, ArH), 7.48(dd, 1H, ArH), 7.59(d, 1H, ArH).

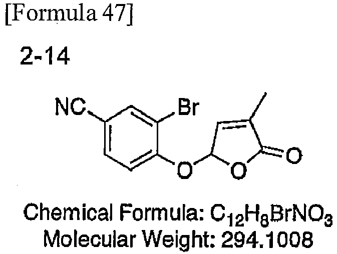

¹H-NMR(CDCl₃, 500 MHz)
d 2.05(s, 3H, CH=CCH₃), 6.31(s, 1H, C=CHCH), 7.08(s, 1H, C=CHCH), 7.40(d, 1H, ArH), 7.64(dd, 1H, ArH), 7.88(d, 1H, ArH).

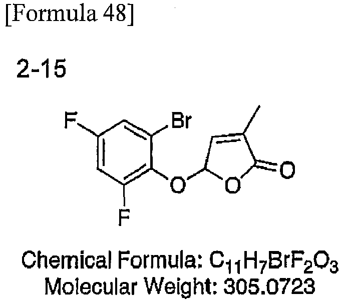

¹H-NMR(CDCl₃, 500 MHz)
d 2.02(s, 3H, CH=CCH₃), 6.21(s, 1H, C=CHCH), 6.92(ddd, 1H, ArH), 7.10(s, 1H, C=CHCH), 7.16(m, 1H, ArH).

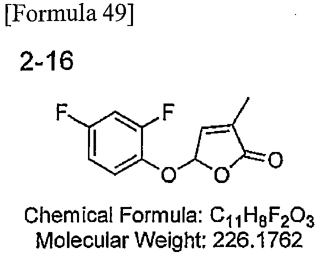

¹H-NMR(CDCl₃, 500 MHz)
d 2.00(s, 3H, CH=CCH₃), 6.20(d, 1H, C=CHCH), 6.82-6.92(m, 2H, ArH), 7.02(d, 1H, C=CH), 7.28 (m, 1H, ArH).

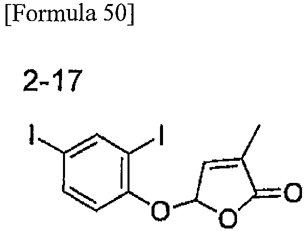

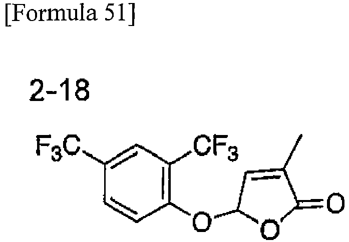

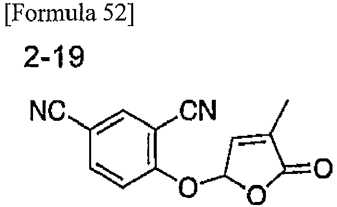

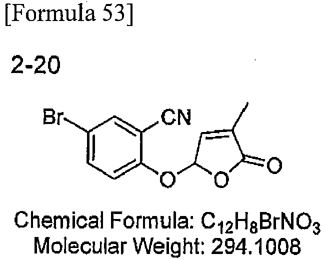

¹H-NMR(CDCl₃, 500 MHz)
d 2.03(s, 3H, CH=CCH₃), 6.31(d, 1H, C=CHCH), 7.15(m, 1H, ArH), 7.26(m, 1H, ArH), 7.89 (dt, 2H, ArH).

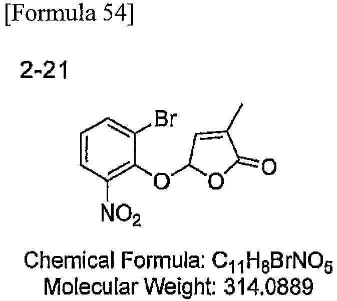

¹H-NMR(CDCl₃, 500 MHz)
d 2.05(s, 3H, CH=CCH₃), 6.30(d, 1H, C=CHCH), 7.09(d, 1H, C=CH), 7.30(d, 1H, ArH), 7.70(d, 1H, ArH), 7.73(d, 1H, ArH).

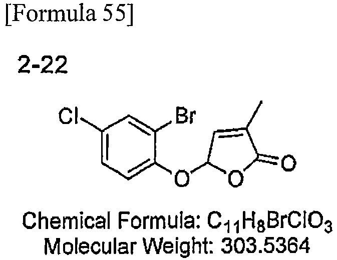

¹H-NMR(CDCl₃, 500 MHz)
d 2.03(s, 3H, CH=CCH₃), 6.19(d, 1H, C=CHCH), 7.06(d, 1H, C=CH), 7.26-7.30(m, 2H, ArH), 7.58(d, 1H, ArH).

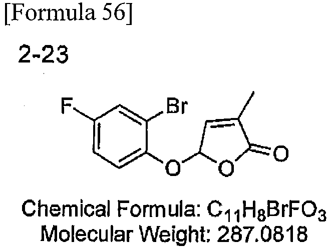

¹H-NMR(CDCl₃, 500 MHz)
d 2.04(s, 3H, CH=CCH₃), 6.35(t, 1H, C=CHCH), 7.03(td, 1H, ArH), 7.07(d, 1H, C=CH), 7.29-7.34(m, 2H, ArH).

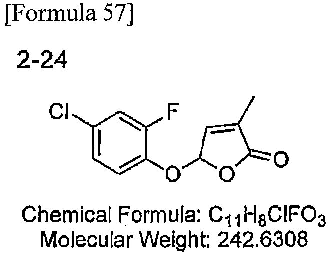

¹H-NMR(CDCl₃, 500 MHz)
d 2.03(s, 3H, CH=CCH₃), 6.23(s, 1H, C=CHCH), 7.02(s, 1H, C=CH), 7.10(d, 1H, ArH), 7.16(d, 1H, ArH), 7.25(d, 1H, ArH).

### [Synthesis Example 6] Synthesis of strigolactone-like compound

Strigolactone-like compounds shown below were synthesized in the same way as in Synthesis Example 1.

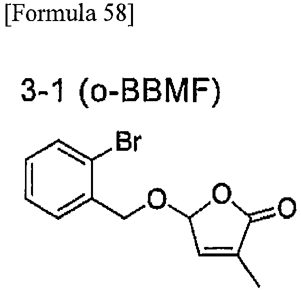

¹H-NMR(CDCl₃, 500 MHz)
d 1.97(s, 3H, CH=CCH₃), 4.83(d, 1H, ArCH), 4.96(d, 1H, ArCH),
5.95(t, 1H, C=CHCH), 6.87(t, 1H, C=CH), 7.20(dt, 1H, ArH), 7.33(t, 1H, ArH), 7.44(d, 1H, ArH), 7.58(d, 1H, ArH).

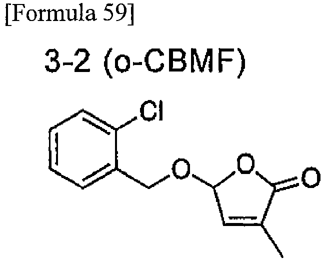

### [Example 1] Tiller (rice branching) inhibitory activity of strigolactone-like compound (1) Effect on rice d10 variant

In this Example, a rice d10 variant (Arite et al., Plant J, 51 (6) 1019-1029 (2007)) was used, and the strigolactone-like compound of the present invention was examined for its effects on this variant. The rice d10 variant exhibits abnormal increase in the number of tillers and a dwarf phenotype, compared with the wild-type. In this d10 variant, the functions of a strigolactone biosynthetic enzyme are inhibited by the variation of the strigolactone biosynthetic gene so that the amount of endogenous strigolactone is decreased.

First, rice seeds were sterilized for 30 minutes with 2.5% sodium hypochlorite containing 0.01% Tween-20 and then washed with sterilized water. Then, the seeds were germinated by culture for 2 days in sterilized water of 25°C under dark conditions. The germinated seeds were transferred to a hydroponic culture medium containing 0.6% agar and grown for 6 days under conditions of a 16-hour light period and an 8-hour dark period under a fluorescent light. Then, the germinated seeds were transferred to a vial containing 12 ml of a sterilized hydroponic culture solution containing each test compound and further grown for 6 days under similar conditions. The number of tillers and the length of each tiller were determined. In this context, the test compounds used were the strigolactone-like compounds BPMF, TFTF, CPMF, NPMF, FPMF, and IPMF shown in Figure 1A, NaMF described in Synthesis Example 3, and GR24 as a control at a concentration of 1 µM, 100 nM, or 10 nM.

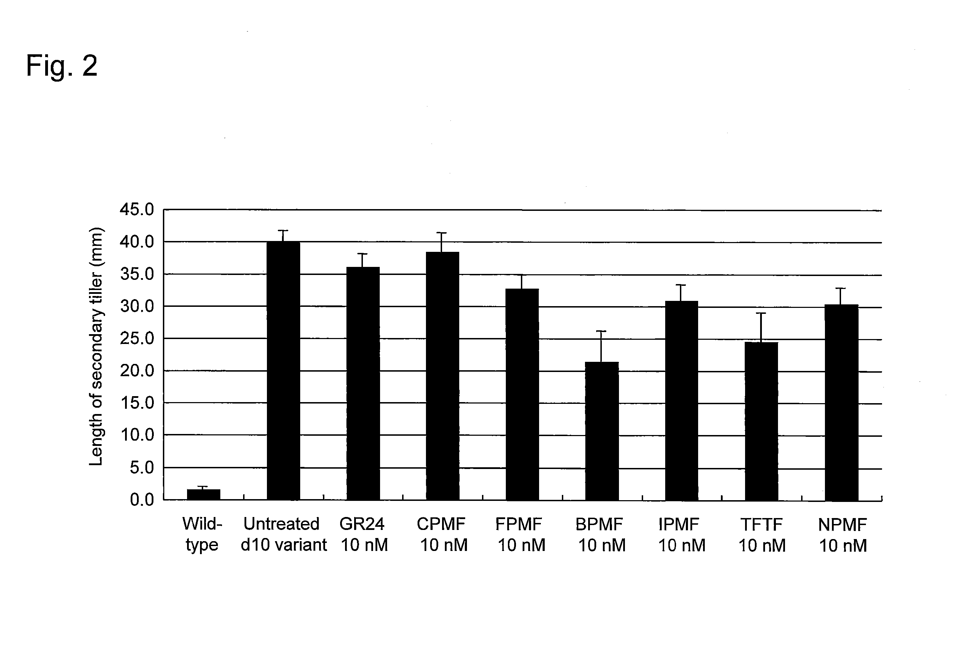

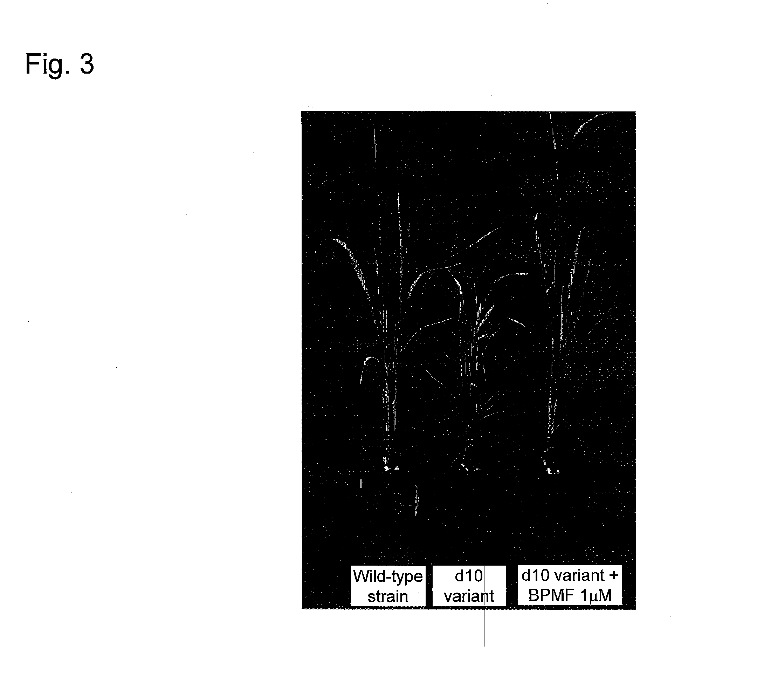

The results are shown in Figures 2 to 4. Figure 2 shows the effects of the strigolactone-like compounds on the rice d10 variant. The strigolactone-like compounds exhibited the activity of inhibiting the morphology of the tiller of the strigolactone-deficient d10 variant. This morphologically inhibitory activity is the typical activity of strigolactone. At the concentration of 10 nM, BPMF exhibited the strongest activity. Also, NaMF exhibited the activity of inhibiting the morphology of the tiller, albeit slightly weaker than that of the other compounds. Figure 3 shows photographs taken after the 12-day growth of wild-type rice, the d10 variant, and the d10 variant treated with 10 µM BPMF. As is evident from Figure 3, the strigolactone-deficient d10 variant restored its morphology similar to that of the wild-type strain.

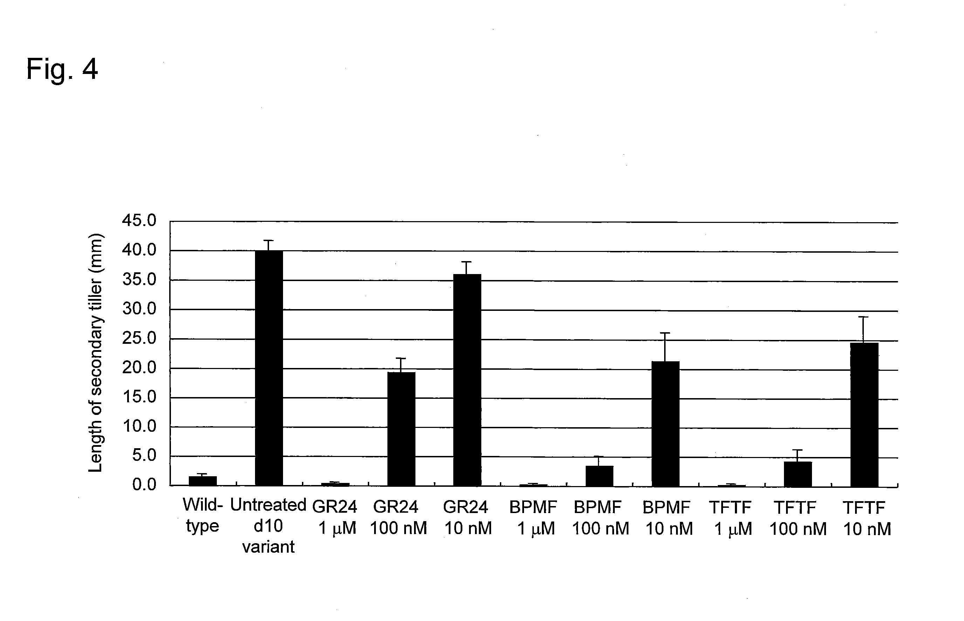

Figure 4 shows the concentration-dependent activities of BPFM, TFTF, and a control compound (GR24) against the rice d10 variant. The length of the secondary tiller observed in the untreated d10 variant was obviously inhibited by treatment with BPMF and TFTF. Particularly, BPMF exhibited strong activity, which was larger than that of the typical non-natural strigolactone GR24.

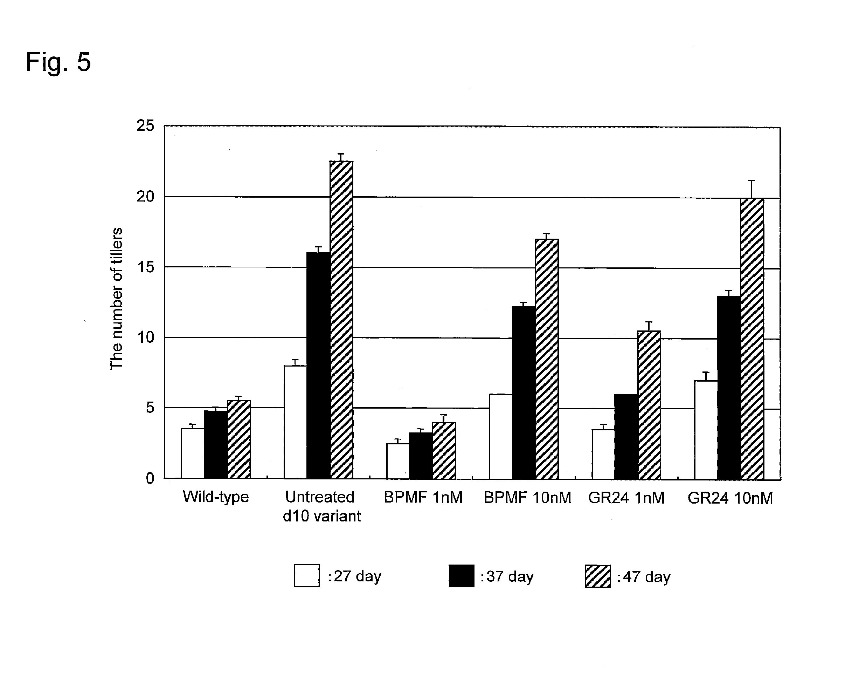

In addition, time-dependent change in the number of tillers was compared between BPMF having the highest activity and GR24. Specifically, the morphology of the tiller of the d10 variant was observed 27 days, 37 days, and 47 days after treatment with 1 nM or 10 nM BPMF or GR24. The results are shown in Figure 5. As shown in Figure 5, BPMF was confirmed to be more effective and have more persistent activity than GR24.

### (2) Effect on Arabidopsis thaliana max3 variant

In this Example, the strigolactone-like compound BPMF of the present invention was examined for its effects on an *Arabidopsis thaliana* max3 variant. The *Arabidopsis thaliana* max3 variant is a strigolactone-deficient variant, as in the rice d10 variant, and is known to have a larger number of branches (Booker J. et al., Current Biology, 14: 1232-1238 (2004)).

Briefly speaking, the surfaces of the respective seeds of wild-type *Arabidopsis thaliana* and the max3 variant were separately sterilized for 5 minutes with a 1% aqueous sodium hypochlorite solution. The thus-treated seeds of each strain were washed five times with sterilized water and allowed to absorb water at 4°C for 1 day under dark conditions. Then, the seeds were inoculated to a 1/2 MS medium containing 1% sucrose and 0.8% agar and cultured at 22°C for 15 days under conditions of a 16-hour day length under a fluorescent light (60 to 70 µmol m⁻² s⁻¹). Subsequently, 400 ml of a hydroponic culture solution described in Noren et al., 2004, Physiologia Plantarum 121: 343-348 was placed in each glass pot covered with aluminum foil, and each test compound was added thereto at a concentration of 5 µM. Nine healthy seedlings were selected from each pot and further hydroponically cultured for 15 days (a total of 30 days) so as to prevent the above-ground part from being wetted by the hydroponic culture solution. The test compounds used were BPMF and GR24 (control). The test compound-containing hydroponic culture solution was replaced every three days.

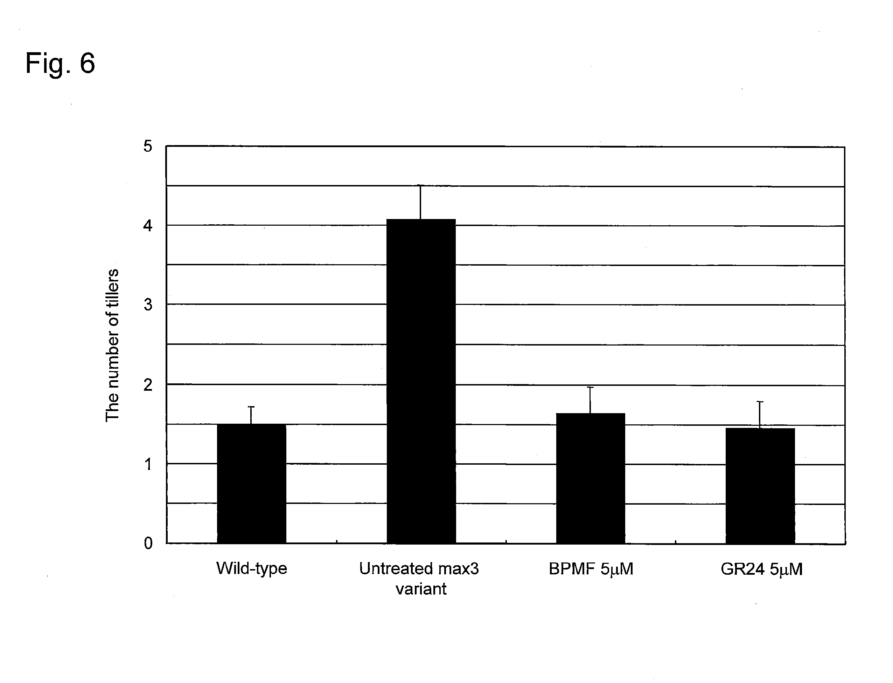

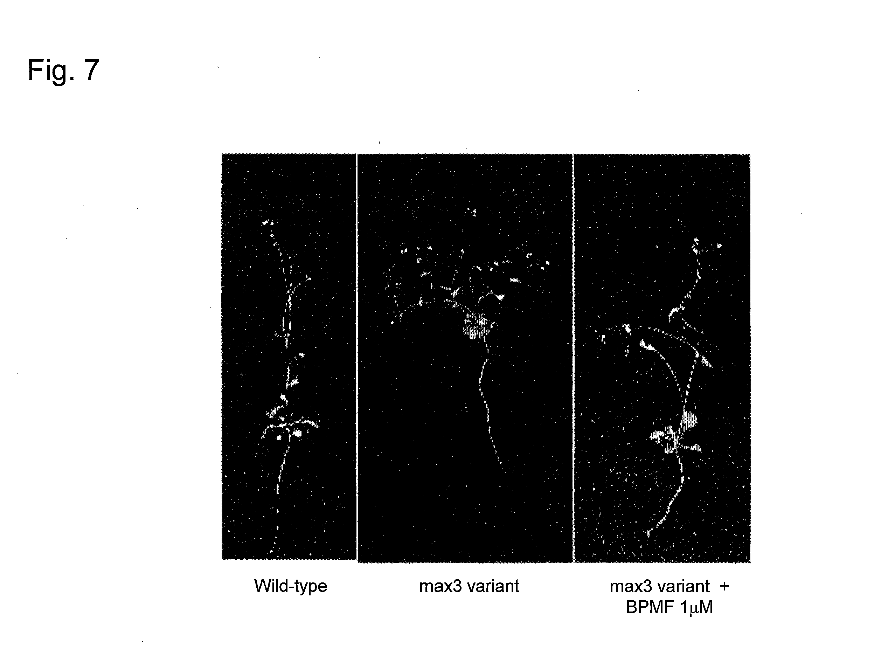

The results are shown in Figures 6 and 7. Figure 6 shows the effects of the strigolactone-like compounds on the *Arabidopsis thaliana* max3 variant. The strigolactone-like compound BPMF and the control GR24 complemented the strigolactone deficiency of the max3 variant and exhibited the activity of inhibiting the morphology of the tiller of the max3 variant. Figure 7 shows photographs taken after the 30-day growth of wild-type +*Arabidopsis thaliana,* the max3 variant, and the max3 variant treated with 1 µM BPMF. As is evident from Figure 7, the strigolactone-deficient max3 variant restored its morphology similar to that of the wild-type strain.

The test results described above demonstrated that the compound of the present invention actually has a strigolactone-like activity and exhibits plant branching inhibitory activity.

### [Example 2] Parasitic weed germination-promoting activity

In this Example, the seeds of a parasitic weed *Orobanche minor* or *Striga hermonthica* were used, and the strigolactone-like compound of the present invention was examined for its parasitic weed germination-promoting activity.

The *Striga hermonthica* seeds were dipped in a 0.5% sodium hypochlorite solution containing 0.03% Tween and sterilized by treatment for 3 minutes using an ultrasonic generator. The seeds were washed three times with sterilized water and surface-dried at 27°C. Then, approximately 50 seeds were left, for preculture, at room temperature for 10 to 12 days under dark conditions on an 8-mm glass fiber filter paper moistened with 50 µl of sterilized water. A solution of each test compound concentration-adjusted by dissolution in sterilized water was used in bioassay. A filter paper was placed on a 5-cm Petri dish, and 1 ml of the test compound solution or sterilized water for comparison was added thereto. The glass fiber filter paper with the precultured parasitic weed seeds placed thereon was put on the resulting Petri dish and cultured at 30°C for 24 hours, followed by calculation of the germination rate. The test compounds used were the strigolactone-like compound BPMF shown in Figure 1A and GR24 as control at a concentration of 50 µM.

Similarly, the *O. minor* seeds were precultured at 23°C for 6 days, treated with each test compound, and then cultured at 23°C for 5 days, followed by calculation of the germination rate. The test compounds used were the strigolactone-like compounds BPMF, CBMF, p-CPMF, m-CPMF, and o-CPMF shown in Figure 1B at a concentration of 1 µM or 10 µM and GR24 as a control at a concentration of 0.0001 µM, 0.0003 µM, 0.001 µM, 0.003 µM, 0.01 µM, 0.03 µM, 0.1 µM, or 1 µM.

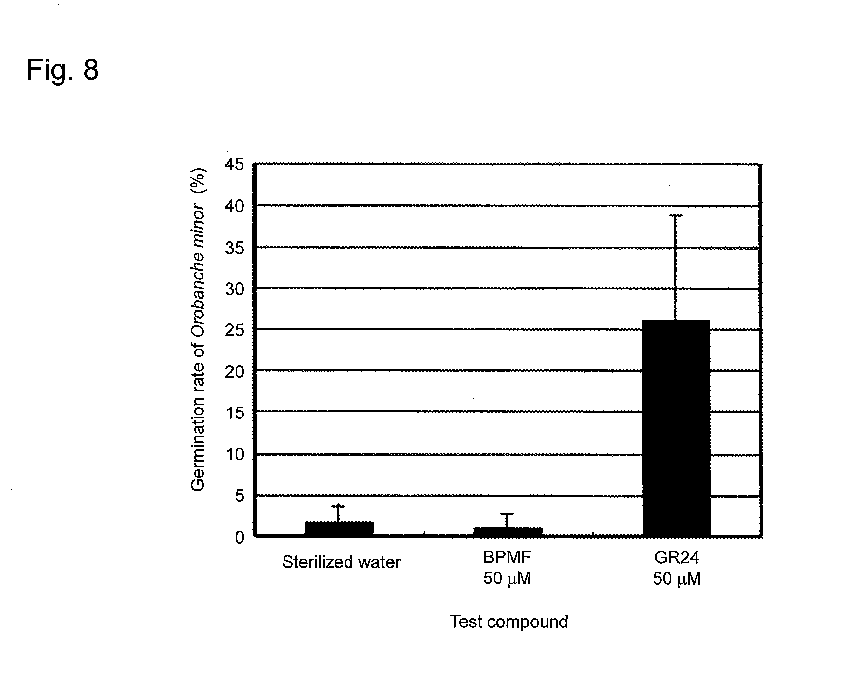

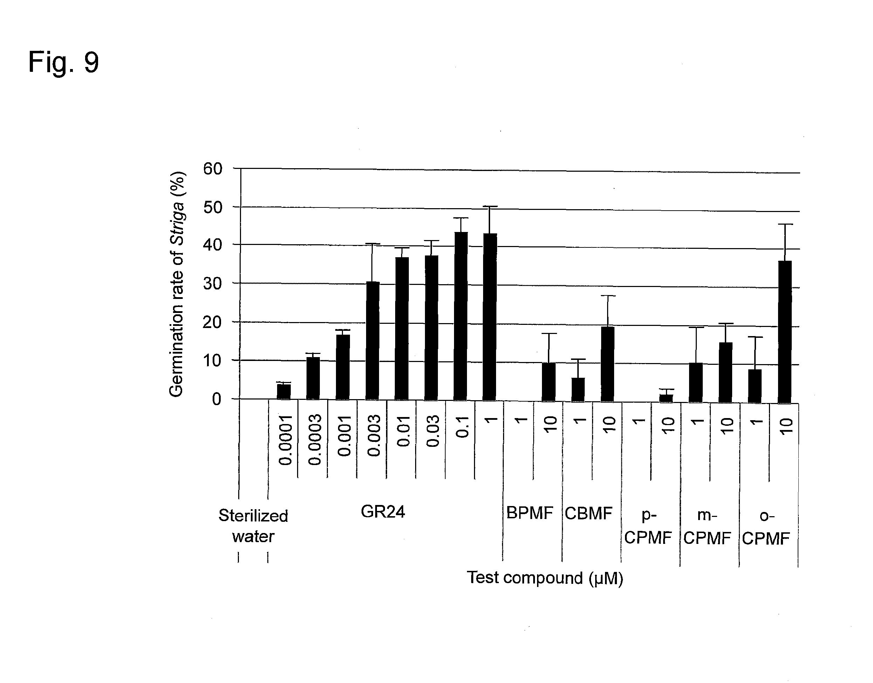

Figures 8 and 9 show the germination rates (%) of the *O. minor* seeds and the *S. hermonthica* seeds, respectively. As shown in Figures 8 and 9, the strigolactone-like compound of the present invention had very weak parasitic weed germination-promoting activity.

The results of Examples 1 and 2 showed no positive correlation between the parasitic weed germination-inducing activity and plant tiller inhibitory activity of the strigolactone-like compound. o- or m-BPMF had weak tiller inhibitory activity, but exhibited stronger parasitic weed germination-inducing activity than that of p-BPMF, though this result is not shown. This compound exhibiting stronger germination-inducing activity may be used as a root parasitic plant control agent by the forced germination of a root parasitic plant.

### [Example 3] Measurement of amount of endogenous strigolactone

In this Example, the amount of strigolactone exudates from a plant treated with the strigolactone-like compound was measured.

A sterilized solution I (2.5% sodium hypochlorite and 0.01% Tween-20) was added to rice seeds (Shiokari) and shaken at room temperature for 15 minutes. The sterilized solution I was discarded, and a sterilized solution II (2.5% sodium hypochlorite) was added thereto and shaken at room temperature for 15 minutes. In a clean bench, the sterilized solution II was discarded, and the seeds were washed five times with sterilized water and left standing at 25°C for 2 days in a dark place. Two days later, the germinated seeds were transplanted in an agar medium for hydroponic rice culture and left standing at 25°C for 6 days under conditions of a 16-hour light period and an 8-hour dark period. Subsequently, 12 mL of a medium for hydroponic rice culture was added to a 12-mL brown vial, and the rice was transplanted therein from the agar medium and left standing at 25°C for 6 days under conditions of a 16-hour light period and an 8-hour dark period. At day 4 during this period, the vial was supplemented with 5 mL of a medium for hydroponic rice culture. The medium for hydroponic rice culture was replaced by a test compound-containing medium for hydroponic culture, and the rice was left standing at 25°C for 24 hours. The test compounds used were the strigolactone-like compound BPMF and GR24 as a control at a concentration of 0.1 µM, 1 µM, or 10 µM.The hydroponic culture solution was directly collected. The root was stored at 4°C in a state dipped in acetone, after measurement of its weight.

To each hydroponic rice culture solution, 200 pg of d₁-2'-epi-5-deoxystrigol per 10 mL of the hydroponic rice culture solution was added, followed by extraction with ethyl acetate (3 mL) twice. The organic layer was dried in nitrogen gas, added to Sep-Pak Silica 1 mL Cartridge (Waters Corp.), and washed with ethyl acetate:n-hexane (15:85), followed by elution with ethyl acetate:n-hexane (35:65). The sample was dried, dissolved in 50% acetonitrile, and analyzed by LC/MS-MS.

To the rice root dipped in acetone, 200 pg of d₁-2'-epi-5-deoxystrigol was added, and the root was then disrupted. The solid matter was filtered and dried in nitrogen gas. Then, ethyl acetate and water were added thereto, followed by extraction with ethyl acetate twice. The organic layer was dried and dissolved in 10% acetone. The solution was added to Oasis HLB 3 mL Cartridge (Waters Corp.) and washed with deionized water, followed by elution with acetone. The organic layer was dried in nitrogen gas, added to Sep-Pak Silica 1 mL Cartridge (Waters Corp.), and washed with ethyl acetate:n-hexane (15:85), followed by elution with ethyl acetate:n-hexane (35:65). The sample was dried, dissolved in 50% acetonitrile, and analyzed by LC/MS-MS.

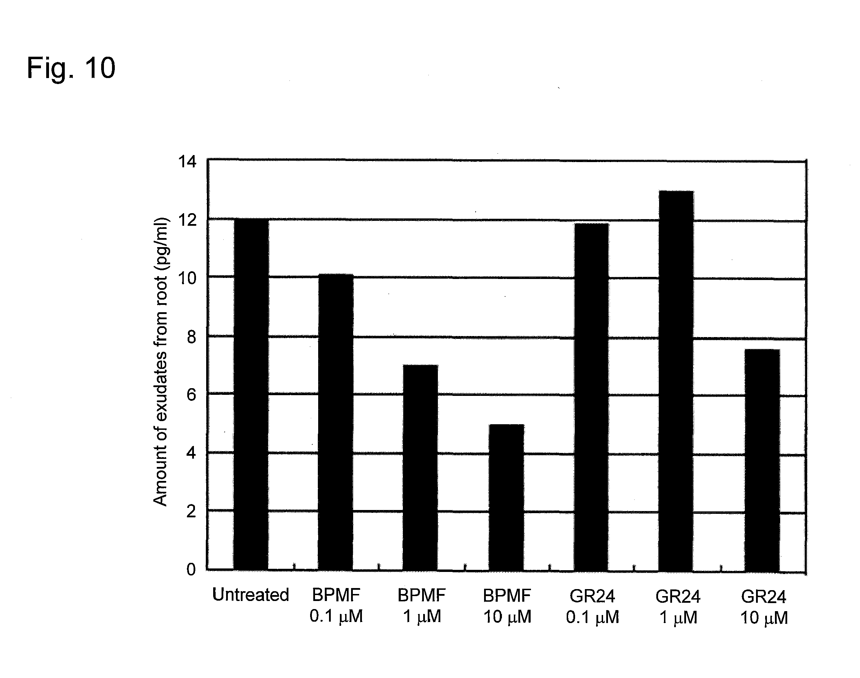

As a result, the amount of plant natural strigolactone synthesized was decreased in the hydroponic rice culture solution and the rice root by the BPMF treatment. Figure 10 shows the results of the amount of endogenous strigolactone exudates from the rice root to the hydroponic culture solution. As shown in Figure 10, the BPMF treatment decreased the amount of natural strigolactone synthesized, and this effect was higher than that of GR24. This decrease is probably due to the feedback effects of non-natural strigolactone on strigolactone biosynthesis. The results of this Example combined with the results of Example 2 suggest that treatment with the strigolactone-like compound (BPMF) of the present invention can inhibit strigolactone biosynthesis and inhibit the germination of a parasitic weed.

### [Example 4] Stability test

The strigolactone-like compound BPMF was left for 30 days in water at 20°C, and change in its activity was then determined. Briefly speaking, rice was hydroponically cultured in the same way as in the hydroponic rice culture solution test conducted in Example 3, and the amount of a residual compound in the resulting solution was calculated using HPLC. First, the hydroponic culture solution adjusted to a predetermined concentration was collected at regular intervals and filtered through a filter. Then, a predetermined amount (e.g., 10 µl) was analyzed by HPLC. The content was determined on the basis of an area value, and the degree of decomposition was calculated by comparison with a standard.

As a result, 95% or more of BPMF was stably present even after being left for 30 days in water under conditions of 20°C. The conventional strigolactone GR24 having an enol ether bond, which is frequently used in research, has a half-life of approximately 10 days in water at 20°C, while the natural strigolactone 5-DS has a half-life of approximately 1.5 days. Therefore, the strigolactone-like compound of the present invention was shown to be superior in stability to the conventional strigolactone compound.

### [Example 5] Parasitic weed germination-promoting activity

In the same way as in Example 2, the strigolactone-like compound was examined for its parasitic weed germination-promoting activity using the seeds of a parasitic weed *Striga hermonthica.* In this context, the test compounds used were TFTF, NPMF, CPMF, BPMF, FPMF, IPMF, MPMF, MSMF, and TBMF at a concentration of 1 µM, 10 µM, or 100 µM and GR24 as a control compound at a concentration of 0.0001 µM, 0.001 µM, 0.01 µM, 0.1 µM, 1 µM, or 10 µM.

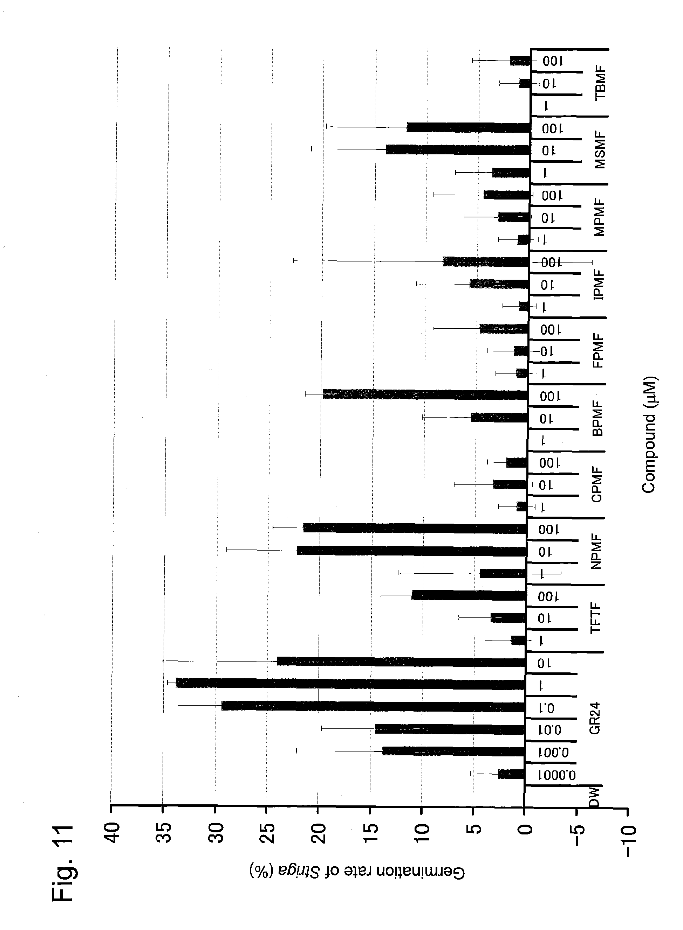

Figure 11 shows the germination rate (%) of the *S. hermonthica* seeds. In the diagram, "DW" represents sterilized water used as a control. As shown in Figure 11, the strigolactone-like compound of the present invention had weak parasitic weed germination-promoting activity.

### [Example 6] Tiller (rice branching) inhibitory activity of strigolactone-like compound

In the same way as in Example 1(1), the strigolactone-like compound was examined for its effects on rice tillers.

In this context, the test compounds used were p-CPMF, m-CPMF, o-CPMF, o-FPMF (1-14), o-BPMF (1-15), p-BPMF, 1-NaphMF (1-16), 2-NaphMF (NaMF), CNMF (1-17), CQMF (1-19), and TeMF (1-18) and GR24 as a control at a concentration of 1 µM or 100 nM. Also, PMF and p-CBMF were used at a concentration of 1 µM.

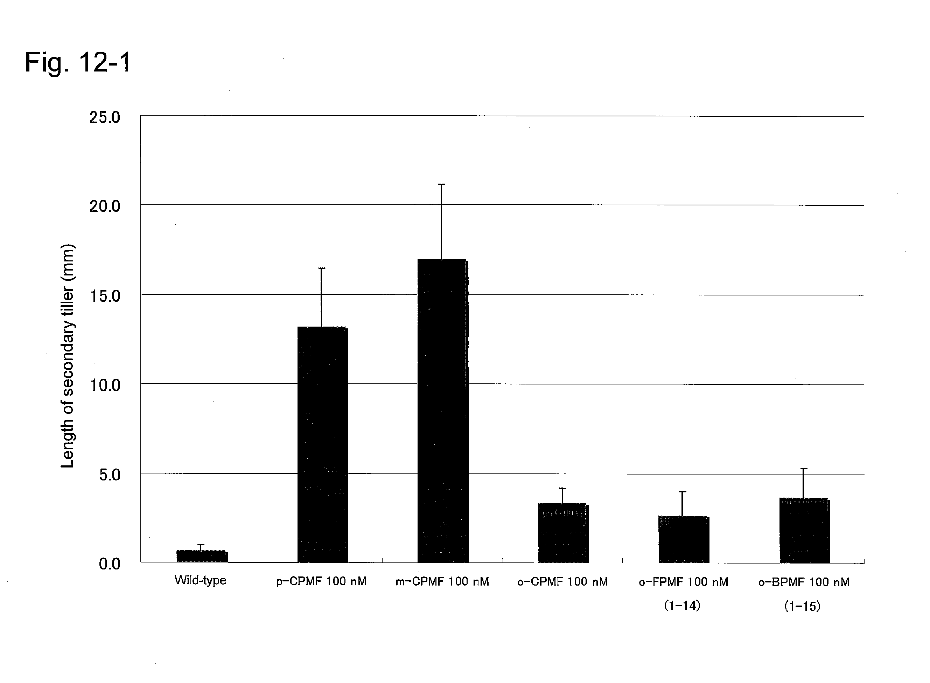

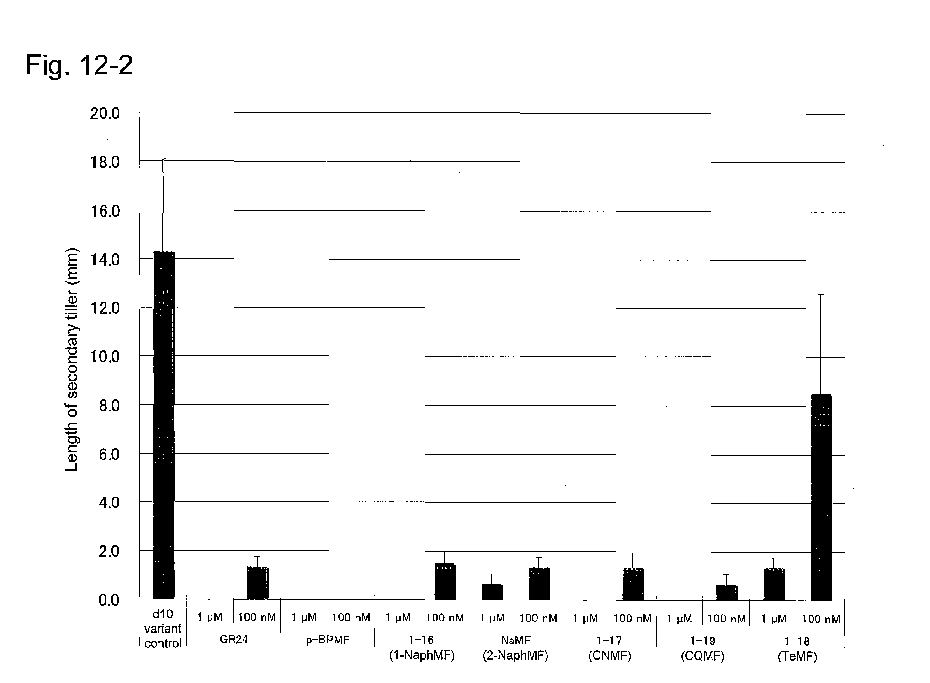

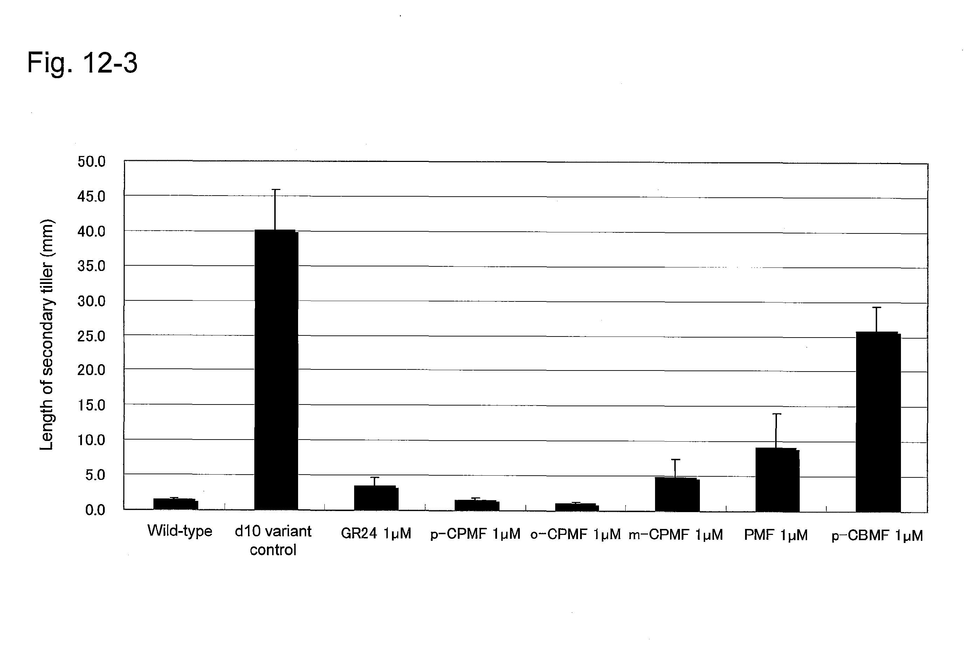

The results are shown in Figures 12-1, 12-2, and 12-3. These diagrams show the effects of the strigolactone-like compounds on the rice d10 variant. The strigolactone-like compounds exhibited the activity of inhibiting the morphology of the tiller of the strigolactone-deficient d10 variant in a concentration-dependent manner. This morphologically inhibitory activity is the typical activity of strigolactone. At the concentration of 100 nM, p-BPMF exhibited the strongest activity.

### [Example 7] Parasitic weed germination-promoting activity

In the same way as in Example 2, the strigolactone-like compound was examined for its parasitic weed germination-promoting activity using the seeds of a parasitic weed *Striga hermonthica.* In this context, the test compounds used were o-CPMF, o-FPMF (1-14), o-BPMF (1-15), o-MSMF (1-20), o-NPMF (1-21), PMF, CQMF (1-19), CNMF (1-17), TeMF (1-18), 2-NaphMF (NaMF), and 1-NaphMF (1-16) at a concentration of 1 µM, 10 µM, or 100 µM and GR24 as a control compound at a concentration of 0.001 µM, 0.01 µM, 0.1 µM, 1 µM, 10 µM, or 100 µM.

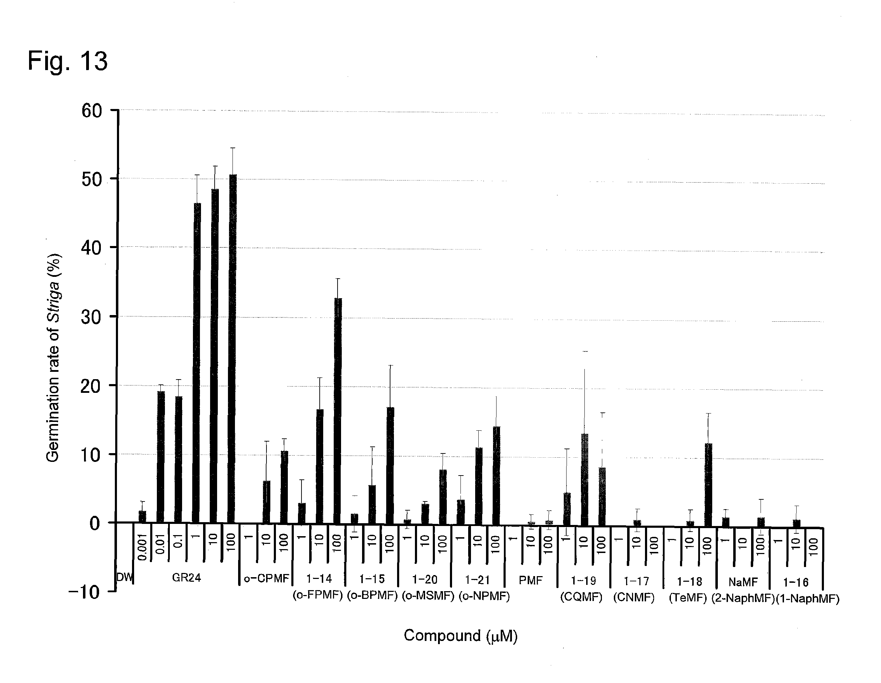

Figure 13 shows the germination rate (%) of the S. *hermonthica* seeds. In the diagram, "DW" represents sterilized water used as a control. As shown in Figure 13, the strigolactone-like compound of the present invention had weak parasitic weed germination-promoting activity.

### [Example 8] Tiller (rice branching) inhibitory activity of strigolactone-like compound

In the same way as in Example 1(1), the strigolactone-like compound was examined for its effects on rice tillers.

In this context, the test compounds used were 2-1 (2,3-DCMF), 2-2 (2,4-DCMF), 2-3 (2,5-DCMF), and 2-4 (2,6-DCMF) at a concentration of 1 µM, 100 nM, or 10 nM and GR24 as a control at a concentration of 1 µM or 100 nM.

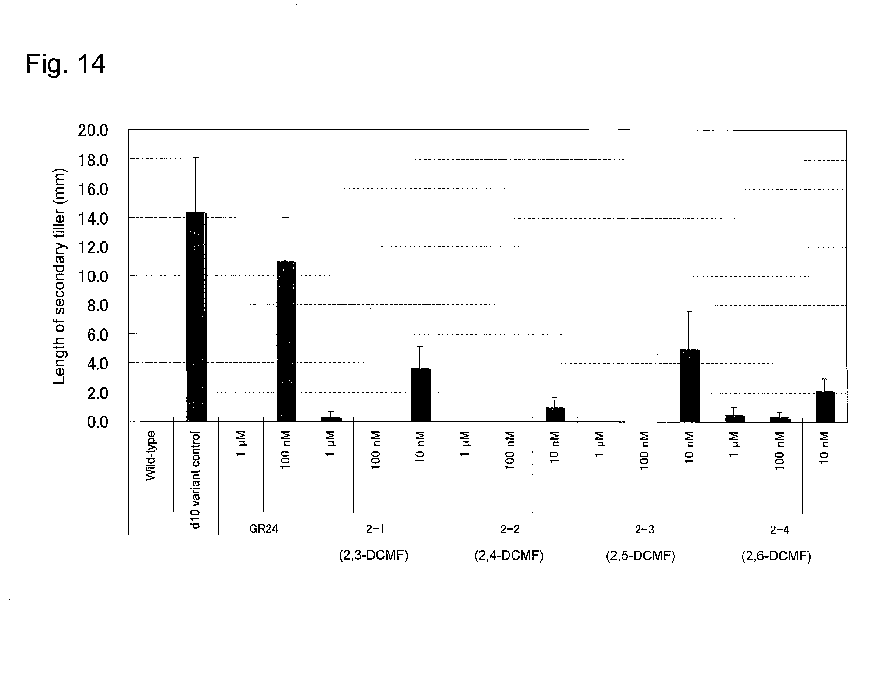

The results are shown in Figure 14. Figure 14 shows the effects of the strigolactone-like compounds on the rice d10 variant. The strigolactone-like compounds exhibited the activity of inhibiting the morphology of the tiller of the strigolactone-deficient d10 variant in a concentration-dependent manner. This morphologically inhibitory activity is the typical activity of strigolactone. At the concentration of 100 nM, 2-1, 2-2, and 2-3 had high activity.

### [Example 9] Parasitic weed germination-promoting activity

In the same way as in Example 2, the strigolactone-like compound was examined for its parasitic weed germination-promoting activity using the seeds of a parasitic weed *Striga hermonthica.* In this context, the test compounds used were BPMF, 2-1, 2-2, 2-3, 2-4, 2-6, 2-7, 2-8, 2-9, 2-10, 2-11, 2-12, 2-13, 2-14, 2-15, 2-16, 2-20, 2-21, 2-22, 2-23, and 2-24 at a concentration of 1 µM, 10 µM, or 100 µM, 2-5 at a concentration of 0.1 µM, 1 µM, 10 µM, or 100 µM, and GR24 as a control compound at a concentration of 0.001 µM, 0.01 µM, 0.1 µM, 1 µM, 10 µM, or 100 µM.

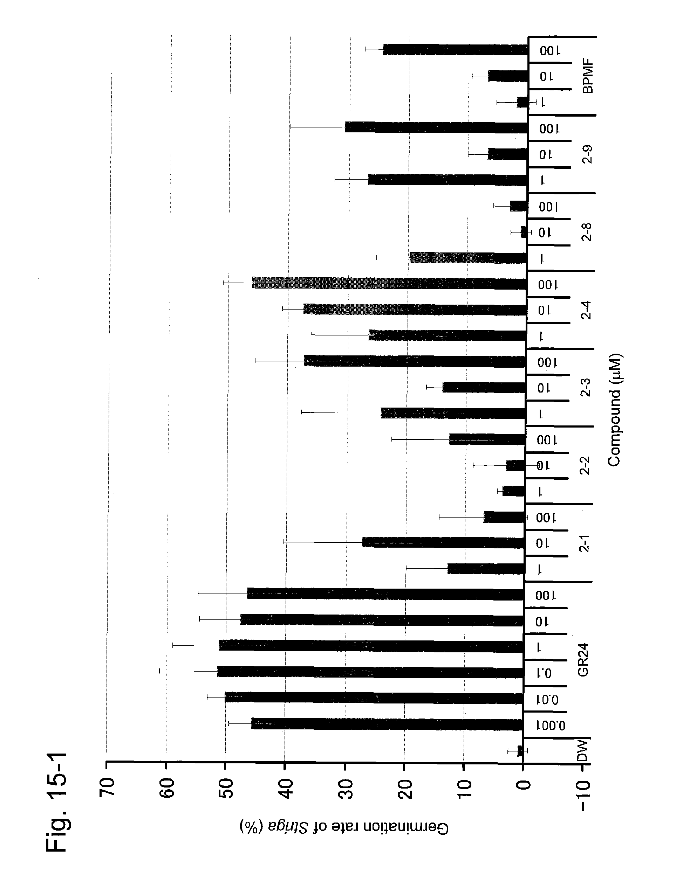

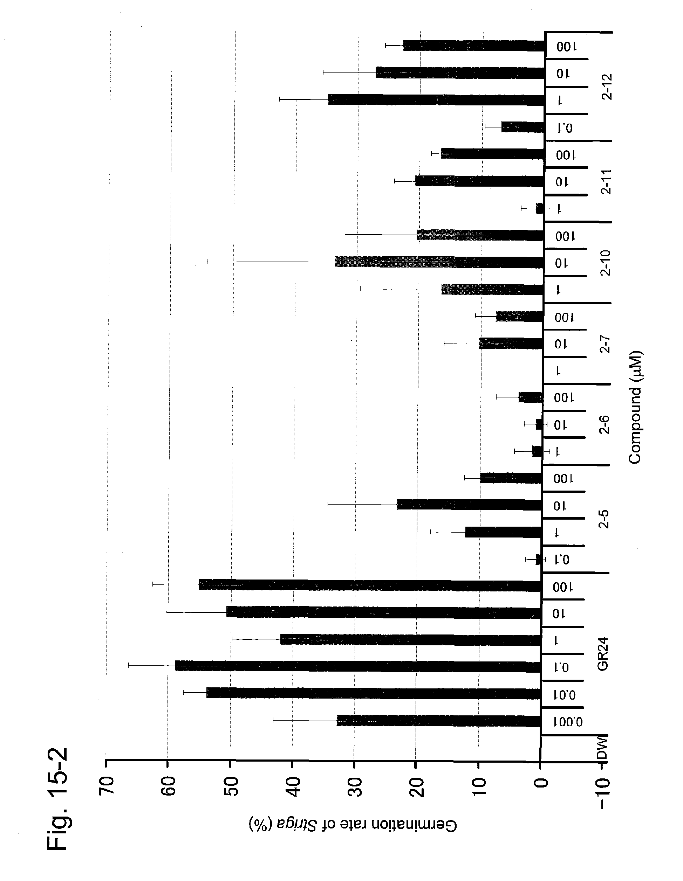

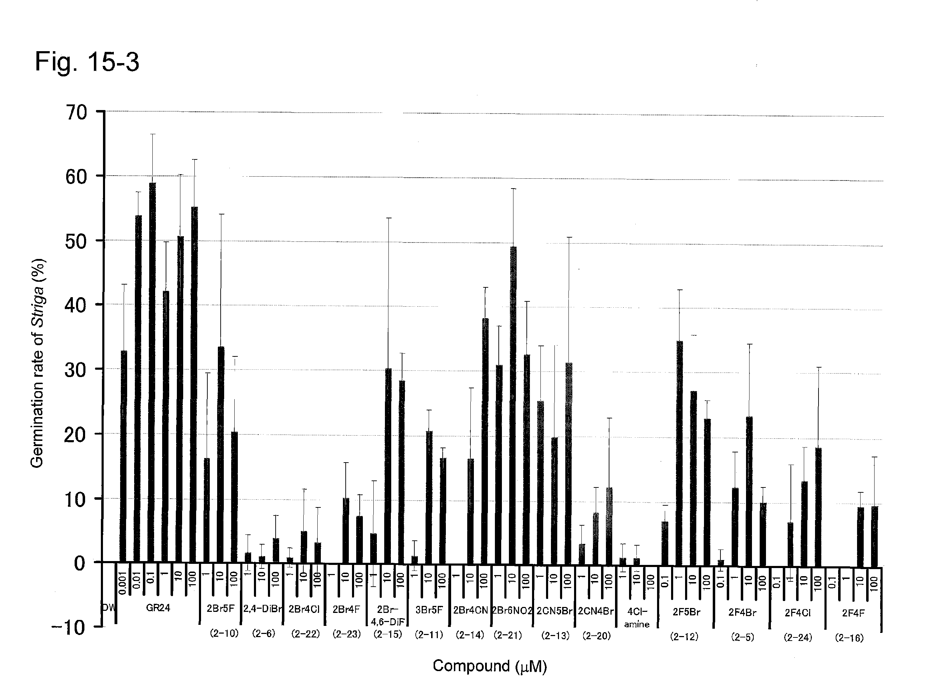

Figures 15-1, 15-2, and 15-3 show the germination rate (%) of the *S. hermonthica* seeds. In the diagrams, "DW" represents sterilized water used as a control. As shown in these diagrams, the strigolactone-like compound of the present invention had weak parasitic weed germination-promoting activity.

### [Example 10] Tiller (rice branching) inhibitory activity of strigolactone-like compound

In the same way as in Example 1(1), the strigolactone-like compound was examined for its effects on rice tillers.

In this context, the test compounds used were p-BPMF, 2,4-DCMF (2-2), o-BBMF (3-1), o-BPMF (1-15), o-CBMF (3-2), o-CPMF, and o-FPMF (1-14) at a concentration of 100 nM or 10 nM, 2-16, 2-6, 2-17, 2-18, and 2-19 at a concentration of 0.1 µM or 1 µM, and GR24 as a control at a concentration of 1 µM or 100 nM.

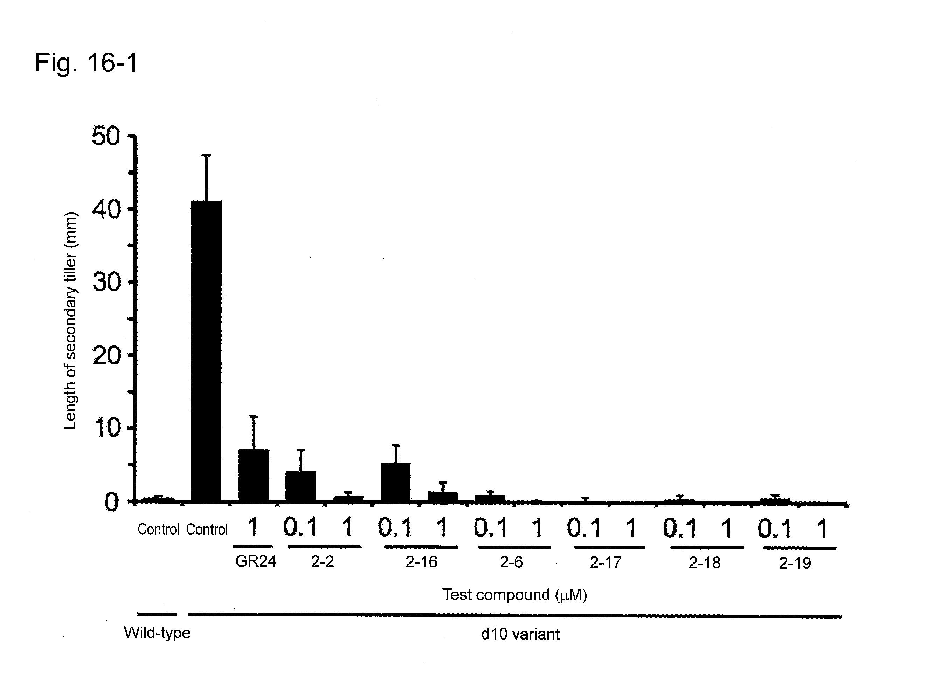

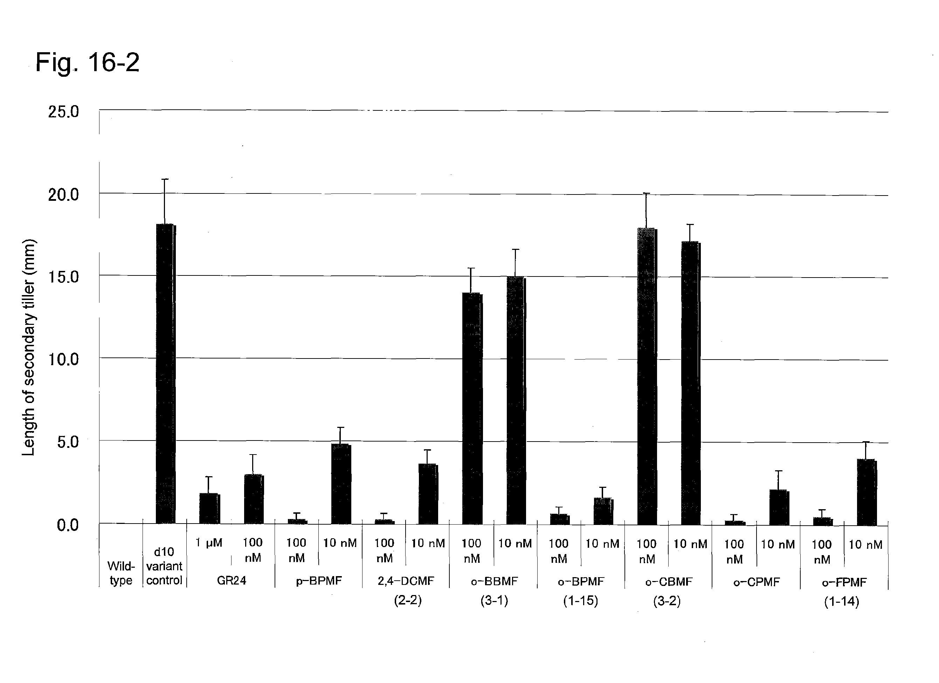

The results are shown in Figures 16-1 and 16-2. These diagrams show the effects of the strigolactone-like compounds on the rice d10 variant. The strigolactone-like compounds exhibited the activity of inhibiting the morphology of the tiller of the strigolactone-deficient d10 variant in a concentration-dependent manner. This morphologically inhibitory activity is the typical activity of strigolactone. At the concentration of 100 nM, p-BPMF, 2,4-DCMF (2-2), o-BPMF (1-15), o-CPMF, o-FPMF, 2-16, 2-6, 2-17, 2-18, and 2-19 had high activity.

All publications, patents, and patent applications cited herein are incorporated herein by reference in their entirety.

### Industrial Applicability

The present invention provides a novel compound that has a strigolactone-like activity and can be chemically synthesized easily. This compound can be used in agricultural chemical compositions such as plant branching inhibitors and root parasitic plant control agents. Thus, the present invention is useful in fields such as agriculture, forestry, pesticide science, and horticultural science.

## Claims

1. A compound represented by the following general formula (III) or a salt thereof: 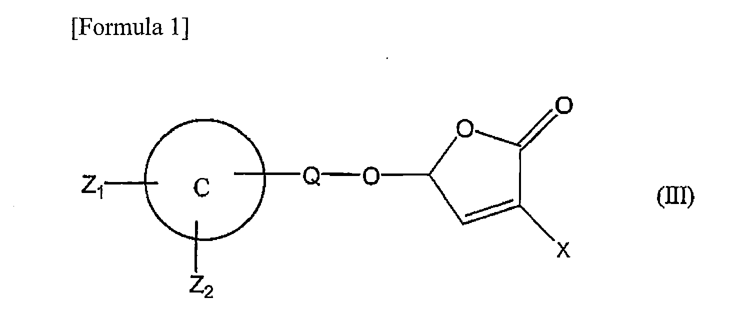 wherein
X represents a lower alkyl group, a lower alkenyl group, a lower alkynyl group, a halogen atom, an OH group, a CN group, a NO₂ group, a CF₃ group, a carboxyl group, or a carboxyl group substituted by a lower alkyl group or a halogen atom;
Q represents a single bond, -CO-, or -CH₂-; and
ring C represents a 6-membered ring represented by the following formula (IVa) or (IVb) or a 5-membered ring represented by the following formula (IVc) or (IVd): 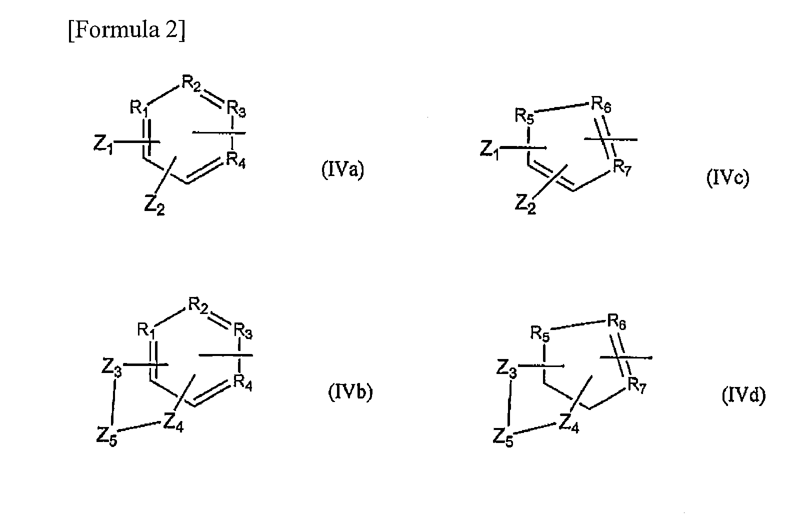 wherein R1, R2, R3, and R4 in the formulas (IVa) and (IVb) each independently represent a carbon atom optionally bonded to a halogen atom or an oxygen atom, a nitrogen atom, a sulfur atom, or an oxygen atom,
R5, R6, and R7 in the formulas (IVc) and (IVd) each independently represent a carbon atom optionally bonded to a halogen atom or an oxygen atom, a nitrogen atom, a sulfur atom, or an oxygen atom,
Z1 and Z2 in the formulas (IVa) and (IVc) are bonded to the ring atoms of ring C and each independently represent a hydrogen atom, a halogen atom, a NO₂ group, a CF₃ group, a CN group, an alkyl group, an alkoxy group, a carboxyl group, a carboxyl group substituted by a lower alkyl group or a halogen atom, an aryl group, an aryloxy group, or an aryl group or an aryloxy group substituted by lower alkyl or a halogen atom,
Z3 and Z4 in the formulas (IVb) and (IVd) each independently represent a carbon atom, a nitrogen atom, a sulfur atom, or an oxygen atom bonded to Z5 to form a 5-membered ring or a 6-membered ring, and Z5 is bonded to Z3 and Z4 to form the 5-membered ring or the 6-membered ring and represents a group constituted of atoms selected from the group consisting of a carbon atom, a nitrogen atom, a sulfur atom, and an oxygen atom.

2. The compound according to claim 1 or a salt thereof, wherein Q is a single bond.

3. The compound according to claim 1 or 2 or a salt thereof, wherein ring C is a 6-membered ring represented by the formula (IVa).

4. The compound according to any one of claims 1 to 3 or a salt thereof, wherein each of R1, R2, R3, and R4, or each of R5, R6, and R7 is a carbon atom or a carbon atom bonded to a halogen atom or an oxygen atom.

5. The compound according to any one of claims 1 to 4 or a salt thereof, wherein ring C is a ring represented by the formula (IVa) or (IVc) wherein Z1 is a hydrogen atom, a halogen atom, a NO₂ group, a CF₃ group, a methoxy group, a COOCH₃ group, a t-butyl group, or a CN group, and Z2 is a hydrogen atom.

6. The compound according to any one of claims 1 to 4 or a salt thereof, wherein ring C is an aryl group, an aryloxy group, or an aryl group or an aryloxy group substituted by lower alkyl or a halogen atom.

7. The compound according to any one of claims 1 to 4 or a salt thereof, wherein ring C is a ring represented by the formula (IVa) or (IVc) wherein Z1 and Z2 each independently represent a halogen atom, a CN group, or a CF₃ group.

8. The compound according to any one of claims 1 to 7 or a salt thereof, wherein X is a methyl group.

9. The compound according to any one of claims 1 to 8 or a salt thereof, wherein the compound or a salt thereof is a compound represented by the following general formula (V) or a salt thereof: 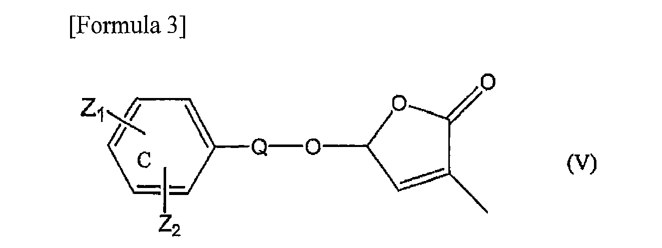 wherein Q represents a single bond or -CO-; and Z1 and Z2 are bonded to the carbon atoms of ring C and each independently represent a hydrogen atom, a halogen atom, a NO₂ group, a CF₃ group, a CN group, an alkyl group, an alkoxy group, a carboxyl group, or a carboxyl group substituted by a lower alkyl group or a halogen atom, or Z1 and Z2 form, together with ring C, an aryl group, an aryloxy group, or an aryl group or an aryloxy group substituted by lower alkyl or a halogen atom.

10. A method for producing a compound represented by formula (III) or a salt thereof, the method comprising the step of reacting a ring C derivative represented by the following general formula (I): 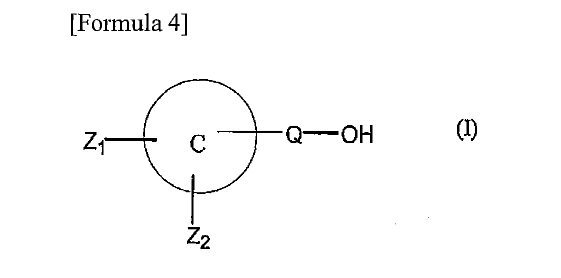 wherein
Q represents a single bond, -CO-, or -CH₂-, and
ring C represents a 6-membered ring represented by the following formula (IVa) or (IVb) or a 5-membered ring represented by the following formula (IVc) or (IVd): 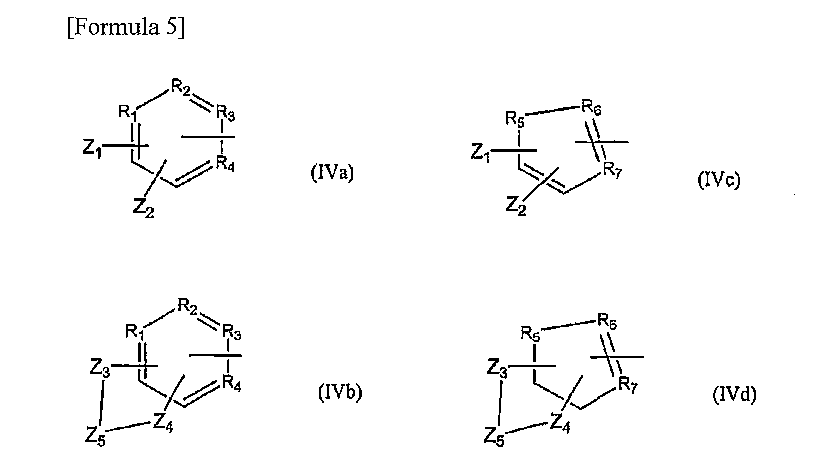 wherein R1, R2, R3, and R4 in the formulas (IVa) and (IVb) each independently represent a carbon atom optionally bonded to a halogen atom or an oxygen atom, a nitrogen atom, a sulfur atom, or an oxygen atom,
R5, R6, and R7 in the formulas (IVc) and (IVd) each independently represent a carbon atom optionally bonded to a halogen atom or an oxygen atom, a nitrogen atom, a sulfur atom, or an oxygen atom,
Z1 and Z2 in the formulas (IVa) and (IVc) are bonded to the ring atoms of ring C and each independently represent a hydrogen atom, a halogen atom, a NO₂ group, a CF₃ group, a CN group, an alkyl group, an alkoxy group, a carboxyl group, a carboxyl group substituted by a lower alkyl group or a halogen atom, an aryl group, an aryloxy group, or an aryl group or an aryloxy group substituted by lower alkyl or a halogen atom,
Z3 and Z4 in the formulas (IVb) and (IVd) each independently represent a carbon atom, a nitrogen atom, a sulfur atom, or an oxygen atom bonded to Z5 to form a 5-membered ring or a 6-membered ring, and Z5 is bonded to Z3 and Z4 to form the 5-membered ring or the 6-membered ring and represents a group constituted of atoms selected from the group consisting of a carbon atom, a nitrogen atom, a sulfur atom, and an oxygen atom,
with a brominated lactone represented by the following general formula (II): 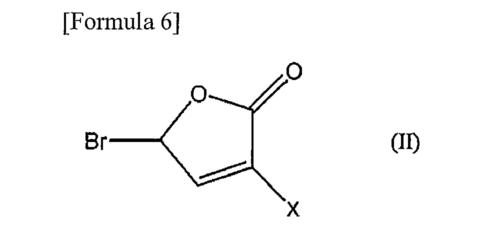 wherein
X represents a lower alkyl group, a lower alkenyl group, a lower alkynyl group, a halogen atom, an OH group, a CN group, a NO₂ group, a CF₃ group, a carboxyl group, or a carboxyl group substituted by a lower alkyl group or a halogen atom,
under basic conditions to obtain a compound represented by the following formula (III): 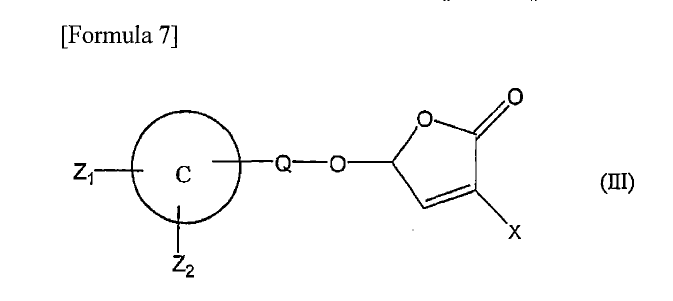 wherein X, Q, ring C, Z1, and Z2 are as defined above.

11. An agricultural chemical composition comprising at least one compound according to any one of claims 1 to 9 and/or a salt thereof and an agriculturally acceptable carrier.

12. The agricultural chemical composition according to claim 11, wherein the agricultural chemical composition is a plant branching inhibitor.

13. The agricultural chemical composition according to claim 12, wherein the plant is a crop plant or a horticultural plant.

14. The agricultural chemical composition according to claim 11, wherein the agricultural chemical composition is a root parasitic plant control agent.

15. The agricultural chemical composition according to claim 14, wherein the root parasitic plant is a plant belonging to the family *Orobanchaceae.*

16. A method for inhibiting the branching of a plant, the method comprising the step of applying at least one compound according to any one of claims 1 to 9 and/or a salt thereof to the plant or a medium or soil for growth of the plant.

17. A method for controlling a root parasitic plant that parasitizes a plant, the method comprising the step of applying at least one compound according to any one of claims 1 to 9 and/or a salt thereof to the plant or a medium or soil for growth of the plant.
